# EUROPÄISCHE PATENTSCHRIFT

(11) **EP 1 185 516 B1**
(45) Veröffentlichungstag und Bekanntmachung des Hinweises auf die Patenterteilung: **02.05.2003**
(21) Anmeldenummer: 00925288.3
(22) Anmeldetag: 16.05.2000
(51) Int. Cl.: C07D 235/14, A61K 31/4184, A61P 9/10, A61P 25/18

(54) **SUBSTITUIERTE PHENYLCYCLOHEXANCARBONSÄUREAMIDE MIT ADENOSINAUFNAHME-HEMMENDER WIRKUNG**
SUBSTITUTED PHENYLCYCLOHEXANE CARBOXYLIC ACID AMIDES THAT HAVE AN ADENOSINE UPTAKE INHIBITING EFFECT
AMIDES D'ACIDE PHENYLCYCLOHEXANECARBOXYLIQUE SUBSTITUES A EFFET INHIBITEUR DE L'ABSORPTION D'ADENOSINE

(30) Priorität: 29.05.1999 DE 19924818
(43) Veröffentlichungstag der Anmeldung: 13.03.2002
(73) Patentinhaber: Bayer Aktiengesellschaft, 51368 Leverkusen (DE)
(72) Erfinder: FREUND, Wolf-Dietrich, D-42799 Leichlingen (DE); LENSKY, Stephen, D-51515 Kürten (DE); MÜLLER, Stephan, Nicholas, D-42349 Wuppertal (DE); PAULSEN, Holger, D-42115 Wuppertal (DE); KELDENICH, Jörg, D-42113 Wuppertal (DE); HORVATH, Ervin, D-51373 Leverkusen (DE); SCHUHMACHER, Joachim, D-42109 Wuppertal (DE)
(86) Internationale Anmeldenummer: EP0004417
(87) Internationale Veröffentlichungsnummer: WO00073275

(56) Entgegenhaltungen:
- EP-A- 0 581 003
- EP-A- 0 582 164
- EP-A- 0 611 767
- EP-A- 0 667 342
- EP-A- 0 725 061
- EP-A- 0 725 064
- US-A- 5 229 505
- US-A- 5 382 584

## Beschreibung

Die vorliegende Erfindung betrifft substituierte Phenylcyclohexancarbonsäureamide mit Adenosinaufnahme-hemmender Wirkung, Verfahren zu ihrer Herstellung und ihre Verwendung in Arzneimitteln, insbesondere zur Behandlung von ischämischen Hirnerkrankungen.

Adenosin ist ein endogener Effektor mit zellprotektiver Wirksamkeit, insbesondere unter zellschädigenden Bedingungen mit begrenzter Sauerstoff- und Substratversorgung wie z.B. bei Ischämie, Hirnschlag und Hirntrauma. Adenosin übt seine neuroprotektive Wirksamkeit im Wesentlichen über eine Unterdrückung präsynaptischer Glutamat-Freisetzung sowie eine Begrenzung postsynaptischer Depolarisation aus. Dadurch wird ein toxischer Calcium-Einstrom in postsynaptische Nervenzellen über NMDA-Rezeptoren verhindert. Die extrazelluläre Konzentration von Adenosin im ZNS erhöht sich dramatisch unter ischämischen bzw. hypoxischen Bedingungen.

Verschiedene Hinweise deuten auf ein neuroprotektives, anticonvulsives, analgetisches und Schlaf-induzierendes Potential von Adenosinaufnahme-Hemmern, da sie die Eigeneffekte von Adenosin durch eine Hemmung seiner zellulären Rückaufnahme verstärken. Deshalb können Adenosinaufnahme-Hemmer in oraler oder intravenöser Applikation zur Vorbeugung und Behandlung von zerebraler Ischämie, Hirnschlag, Reperfusionsschäden, Himtrauma, Ödemen, Krämpfen, Epilepsie, Atemstillstand, Herzstillstand, Reye-Syndrom, zerebraler Thrombose, Embolie, Tumoren, Blutungen, Enzephalomyelitis, Hydroenzephalitis, Rückenmarksverletzungen, post-operative Hirnschäden, Verletzungen der Retina oder des optischen Nervs nach Glaukom, Ischämie, Hypoxie, Ödem oder Trauma sowie in der Behandlung von Schizophrenie, Schlafstörungen und Schmerz eingesetzt werden (*Cerebrovasc. Brain Metab. Rev.* **1992,** *4,* 364-369; *Drug Dev. Res.* **1993,** *28,* 410-415; *Science* **1997**, *276,* 1265-1268; '*Adenosine in the Nervous System',* Ed.: Trevor Stone, Academic Press Ltd. 1991, 217-227; *Ann. Rep. Med*. *Chem*. **1998**, 33, 111-120).

Adenosinaufnahme-Hemmer können auch zur Potenzierung der Wirkung von Nukleobase-, Nukleosid- oder Nukleotid-Antimetaboliten in der chemotherapeutischen Behandlung von Krebs und in der antiviralen (z.B. HIV) Chemotherapie eingesetzt werden (*Curr. Med. Chem.* **1997**, *4*, 35-66).

Aus der EP-A-0 611 767 und der EP-A-0 725 064 sind Phenylcyclohexylcarbonsäureamide bekannt, die zur Behandlung von Atherosklerose bzw. Restenose eingesetzt werden können.

Die vorliegende Erfindung betrifft Verbindungen der allgemeinen Formel (I), 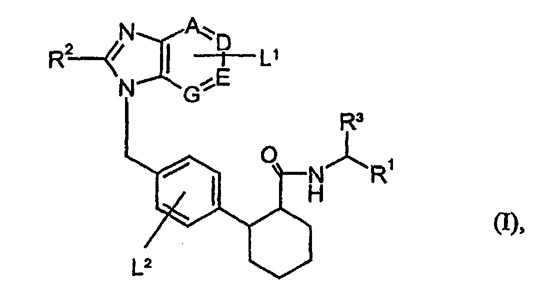 in welcher
A, D, und E jeweils für die CH-Gruppe
G für ein stickstoffatom oder für die CH-gruppe steht,
L¹ und L² für Wasserstoff stehen,
R¹ für einen Rest der Formel CO-NR⁴R⁵ steht,
   worin
   R⁴ und R⁵ Wasserstoff bedeuten,
R² für (C₁-C₄)-Alkyl steht, das gegebenenfalls durch ein Sauerstoff atom unterbrochen ist, oder für 4-R⁷-Piperazin-1-ylrest steht,
   wobei (C₁-C₄)-Alkyl, das gegebenenfalls durch ein Sauerstoff atom unterbrochen ist, durch eine Hydroxyl-Gruppe oder durch einen Rest der Formel -NR⁸R⁹ substituiert ist,
   worin
   R⁷ Wasserstoff, (C₁-C₄)-Alkyl, oder (C₃-C₆)-Cycloalkyl bedeutet,
   R⁸ und R⁹ gleich oder verschieden sind und Wasserstoff, (C₁-C₄)-Alkyl oder (C₃-C₆)-Cycloalkyl bedeuten, oder R⁸ und R⁹ gemeinsam mit dem Stickstoffatom einen Morpholinrest bilden, und R³ für einen Phenylrest steht,
und deren Salze.

Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Die erfindungsgemäßen Verbindungen der allgemeinen Formel (I) können in verschiedenen stereoisomeren Formen auftreten, die sich entweder wie Bild und Spiegelbild (Enantiomere), oder die sich nicht wie Bild und Spiegelbild (Diastereomere) verhalten. Die Erfindung betrifft sowohl die Enantiomeren als auch die Diastereomeren sowie deren jeweilige Mischungen. Die Racemformen lassen sich ebenso wie die Diastereomeren in bekannter Weise in die stereoisomer einheitlichen Bestandteile trennen.

Weiterhin können bestimmte Verbindungen in tautomeren Formen vorliegen. Dies ist dem Fachmann bekannt, und derartige Verbindungen sind ebenfalls vom Umfang der Erfindung umfaßt.

(C₁-C₄)-Alkyl, steht für einen geradkettigen oder verzweigten Alkylrest mit 1 bis 4 Kohlenstoffatomen. Beispielsweise seien genannt: Methyl, Ethyl, n-Propyl, Isopropyl, tert.Butyl, Bevorzugt ist ein geradkettiger oder verzweigter Alkylrest mit 1 bis 3 Kohlenstoffatomen (C₁-C₃).

(C₁-C₄-Alkyl, das durch ein Sauerstoff- latom unterbrochen ist, und das durch eine Hydroxyl-Gruppe oder durch einen Rest der Formel -NR⁸R⁹ substituiert ist, steht beispielsweise für 1,3-Dihydroxy-prop-2-oxy-methyl, 2-Hydroxy-ethoxy-methyl, 2-Hydroxy-prop-1-oxy-methyl, 3-Hydroxy-prop-1-oxy-methyl, Morpholin-4-yl-methyl, 2-Amino-ethyl, 2-Dimethylamino-ethyl, oder Diethylamino-methyl.

(C₃-C₆)-Cycloalky. steh im Rahmen der Erfindung für Cyclopropyl, Cyclobutyl, Cyclopentyl, Cyclohexyl, Bevorzugt seien genannt: Cyclopropyl, Cyclopentyl und Cyclohexyl.

Halogen steht im Rahmen der Erfindung im allgemeinen für Fluor, Chlor, Brom und Jod. Bevorzugt sind Fluor, Chlor und Brom. Besonders bevorzugt sind Fluor und Chlor.

Die Verbindungen der allgemeinen Formel (I) mit der in der allgemeinen Formel (I') angegebenen absoluten Konfiguration

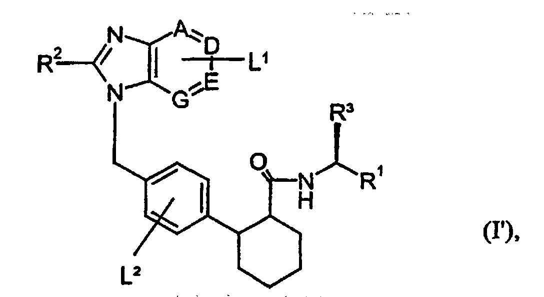

Die erfindungsgemäßen Verbindungen können in vier unterschiedlichen relativen Konfigurationen (A) bis (D) vorliegen:

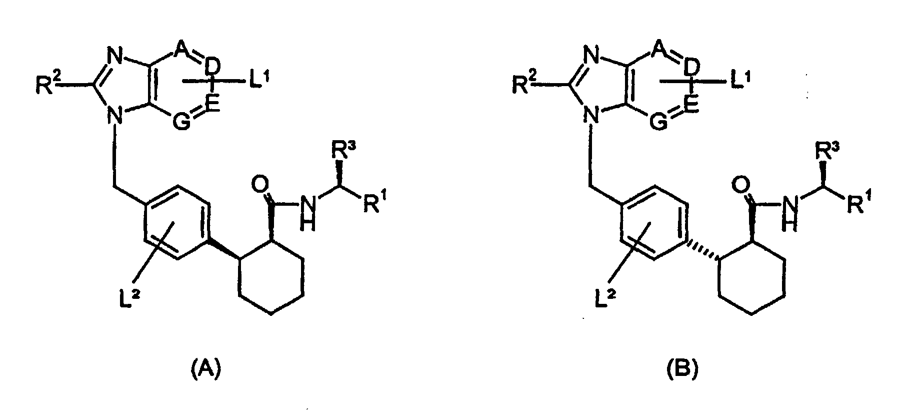

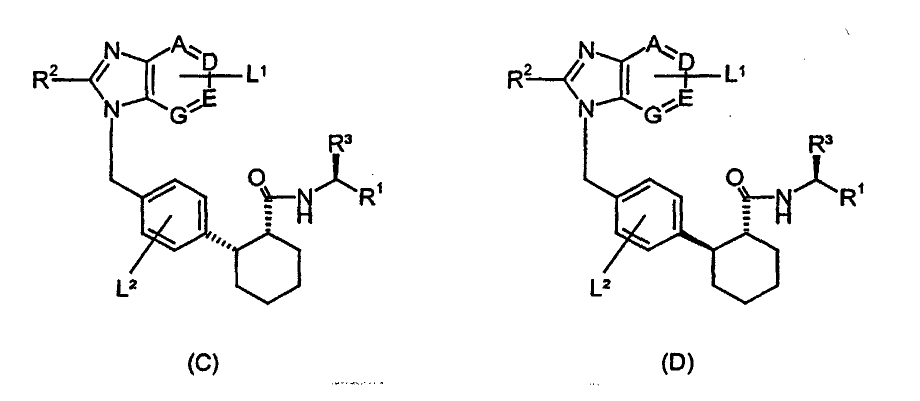

Bevorzugt ist die Konfiguration (D).

Außerdem wurden Verfahren zur Herstellung der erfindungsgemäßen Verbindungen der allgemeinen Formel (I) gefunden, dadurch gekennzeichnet, daß man

### [A] Verbindungen der allgemeinen Formel (II)

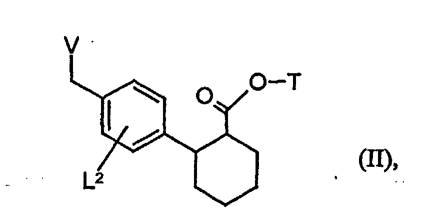

in welcher
L² die oben angegebene Bedeutung hat,
T für (C₁-C₄)-Alkyl, vorzugsweise für Methyl oder tert.Butyl steht,
   und
V für eine geeignete Abgangsgruppe, wie beispielsweise Halogen, Mesylat oder Tosylat, vorzugsweise für Brom steht,
zunächst durch Umsetzung mit Verbindungen der allgemeinen Formel (III) 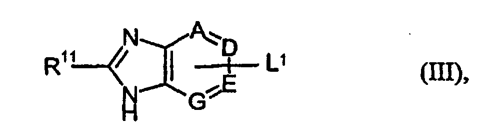 in welcher
A, D, E, G und L¹ die in Anspruch 1 angegebene Bedeutung haben
   und
R¹¹ die in Anspruch 1 aufgeführte Bedeutung von R² hat, wobei Amino- und Hydroxyfunktionen gegebenenfalls durch geeignete Amino- bzw. Hydroxyschutzgruppen blockiert sind,
in inerten Lösemitteln, in Abhängigkeit der Definition von R¹¹ gegebenenfalls in Anwesenheit einer Base in die Verbindungen der allgemeinen Formel (IV) 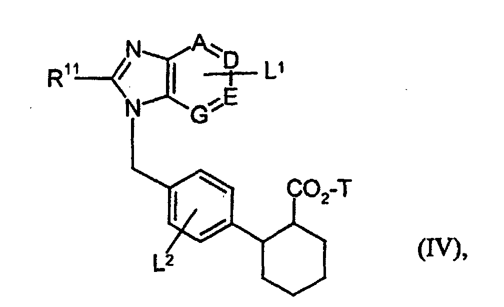 in welcher
R¹¹, A, D, E, G, L¹, L², und T die oben angegebene Bedeutung haben,
überführt,
in einem nächsten Schritt mit Säuren oder Basen in die entsprechenden Carbonsäuren der allgemeinen Formel (V) 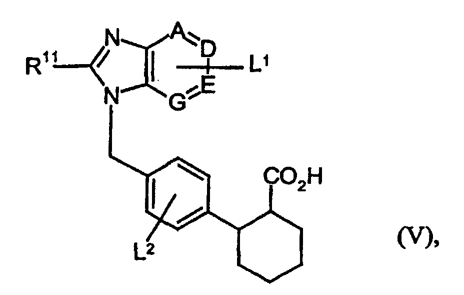 in welcher
R¹¹, A, D, E, G, L¹ und L² die oben angegebene Bedeutung haben,
überführt,
und abschließend nach Aktivierung nach bekannten Methoden mit Verbindungen der allgemeinen Formel (VI) 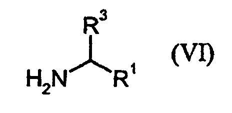 in welcher
R¹ und R³ die oben angegebene Bedeutung haben,
in inerten Lösemitteln umsetzt,
und gegebenenfalls im Fall daß R¹¹ eine der oben aufgeführten Schutzgruppen trägt, diese entweder bei der Hydrolyse zu den Säuren (IV) -> (V) oder nach Umsetzung mit den Verbindungen der allgemeinen Formel (VI) nach üblichen Methoden abspaltet,
oder

### [B] im Fall, daß R² für einen direkt über ein Stickstoffatom an den Imidazolring gebundenen, gesättigten Heterocyclus steht, zunächst die oben aufgeführten Verbindungen der allgemeinen Formel (II) mit Verbindungen der allgemeinen Formel (IIIa)

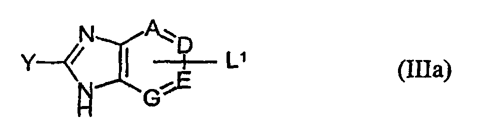

in welcher
A, D, E, G und L¹ die oben angegebene Bedeutung haben
   und
Y für Halogen oder Mesyl, vorzugsweise für Chlor, Brom oder Mesyl steht,
in inerten Lösemitteln in die entsprechenden Verbindungen der Formel (VII) 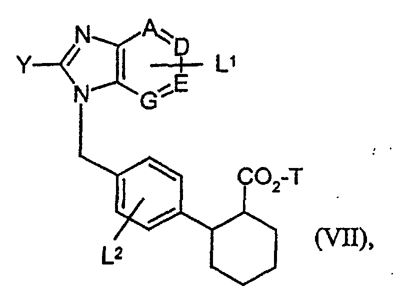 in welcher
Y, A, D, E, G, L¹, L² und T die oben angegebene Bedeutung haben,
überführt,
in einem nächsten Schritt mit Verbindungen der allgemeinen Formel (VIII)

HNR¹²R¹³ (VIII),

in welcher
R¹² und R¹³ zusammen mit dem Stickstoffatom einen Heterocyclus gemäß der Definition von R² bilden,
zu Verbindungen der allgemeinen Formel (IX) 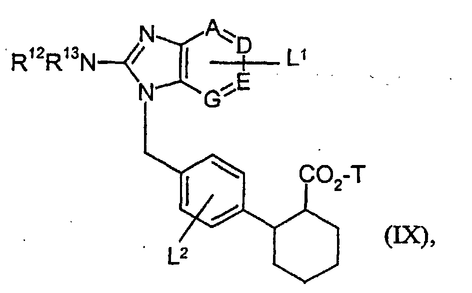 in welcher
A, D, E, G, L¹, L², R¹², R¹³ und T die oben angegebene Bedeutung haben,
umsetzt,
- in den nächsten Schritten, wie unter [A] beschrieben durch Hydrolyse in die entsprechenden Carbonsäuren der allgemeinen Formel (X) 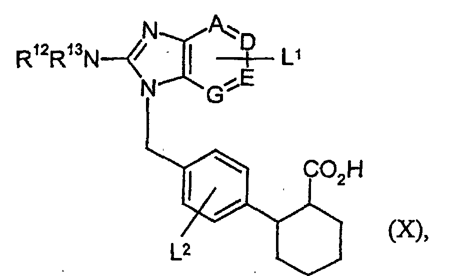 in welcher
A, D, E, G, L¹, L², R¹² und R¹³ die oben angegebene Bedeutung haben,
überführt,
und abschließend nach Aktivierung nach bekannten Methoden zur Herstellung von Amiden aus Carbonsäuren und Aminen mit den Verbindungen der allgemeinen Formel (VI) umsetzt und gegebenenfalls durch Umsetzung mit einer Säure in die entsprechenden Salze überführt.

Die erfindungsgemäßen Verfahren können durch folgende Formelschemata beispielhaft erläutert werden:

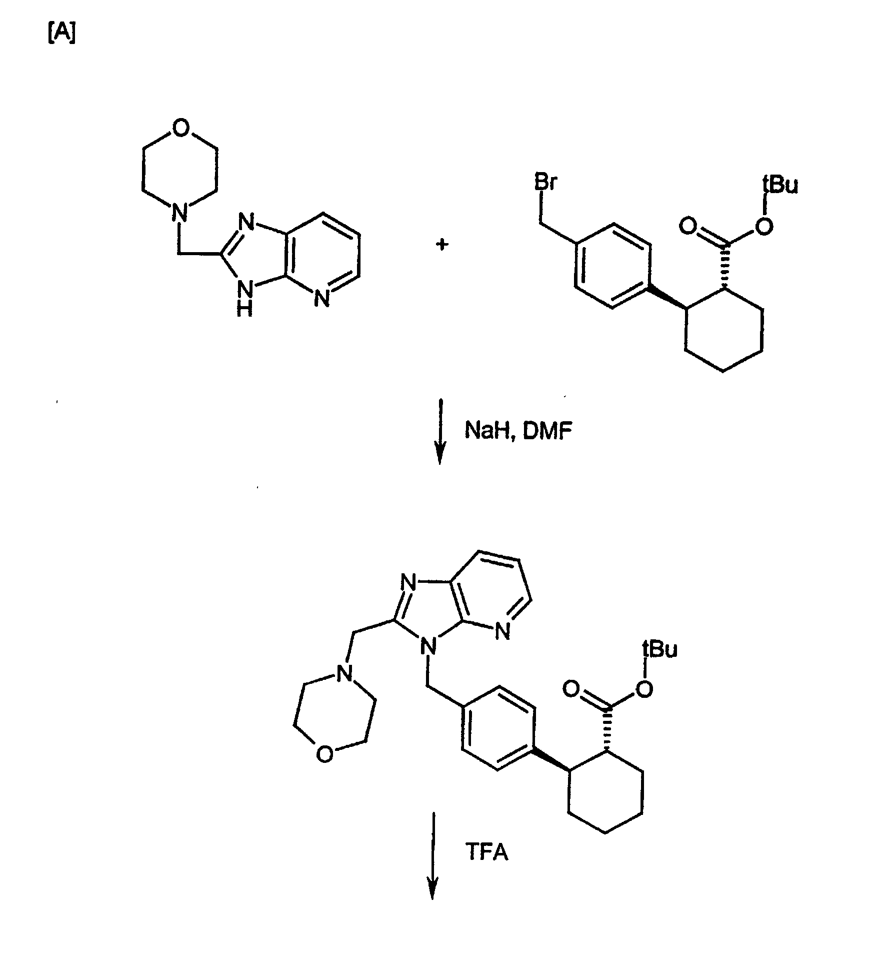

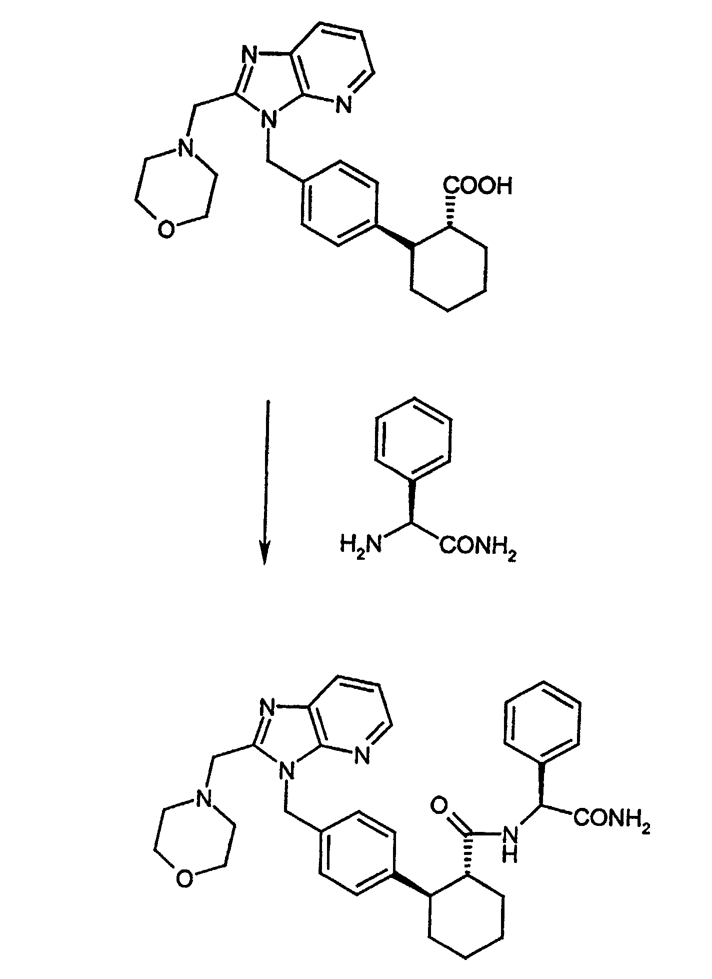

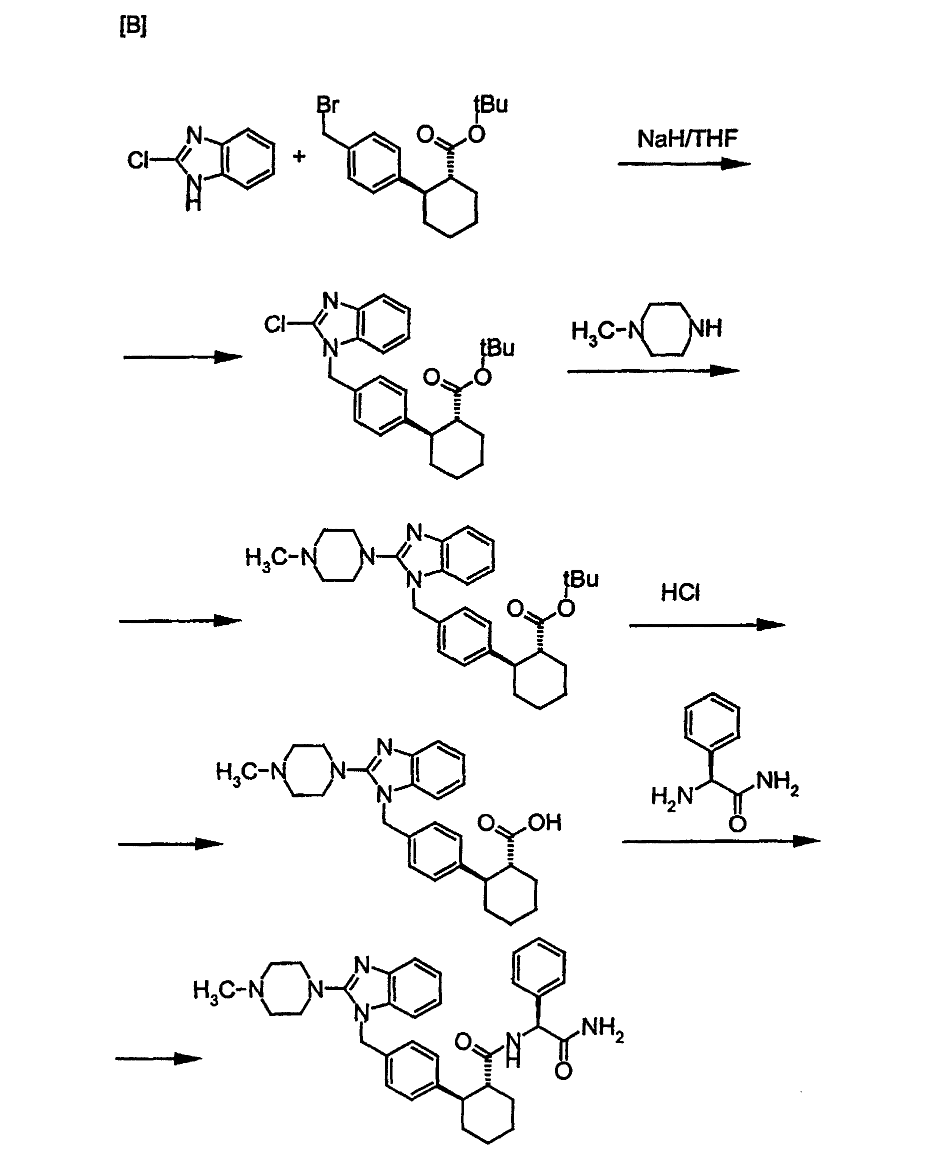

Geeignete Aminoschutzgruppen im Rahmen der Erfindung sind die üblichen in der Peptid-Chemie verwendeten Aminoschutzgruppen.

Hierzu gehören bevorzugt: Benzyloxycarbonyl, 3,4-Dimethoxybenzyloxycarbonyl, 3,5-Dimethoxybenzyloxycarbonyl, 2,4-Dimethoxybenzyloxycarbonyl, 4-Methoxybenzyloxycarbonyl, 4-Nitrobenzyloxycarbonyl, 2-Nitrobenzyloxycarbonyl, 2-Nitro-4,5-dimethoxybenzyloxycarbonyl, Methoxycarbonyl, Ethoxycarbonyl, Propoxycarbonyl, Isopropoxycarbonyl, Butoxycarbonyl, Isobutoxycarbonyl, tert.Butoxycarbonyl, Allyloxycarbonyl, Vinyloxycarbonyl, 2-Nitrobenzyloxycarbonyl, 3,4,5-Trimethoxybenzyloxycarbonyl, Cyclohexoxycarbonyl, 1,1-Dimethylethoxycarbonyl, Adamantylcarbonyl, Phthaloyl, 2,2,2-Trichlorethoxycarbonyl, 2,2,2-Trichlor-tertbutoxycarbonyl, Menthyloxycarbonyl, Phenoxycarbonyl, 4-Nitrophenoxycarbonyl, Fluorenyl-9-methoxycarbonyl, Formyl, Acetyl, Propionyl, Pivaloyl, 2-Chloracetyl, 2-Bromacetyl, 2,2,2-Trifluoracetyl, 2,2,2-Trichloracetyl, Benzoyl, 4-Chlorbenzoyl, 4-Brombenzoyl, 4-Nitrobenzoyl, Phthalimido, Isovaleroyl oder Benzyloxymethylen, 4-Nitrobenzyl, 2,4-Dinitrobenzyl oder 4-Nitrophenyl. Eine bevorzugte Schutzgruppe für primäre Amine ist Phthalimid. Bevorzugte Schutzgruppen für sekundäre Amine sind Benzyloxycarbonyl und tert.Butoxycarbonyl.

Die Abspaltung der Aminoschutzgruppen erfolgt in an sich bekannter Weise, indem man beispielsweise unter hydrogenolytischen, sauren oder basischen Bedingungen, bevorzugt mit Säuren, wie beispielsweise Chlorwasserstoffsäure oder Trifluoressigsäure in inerten Lösemitteln wie Ether, Dioxan und Methylenchlorid arbeitet.

Eine geeignete Hydroxyschutzgruppe im Rahmen der oben angegebenen Definition steht im allgemeinen für eine Schutzgruppe aus der Reihe: Trimethylsilyl, Triethylsilyl, Triisopropylsilyl, tert.Butyl-dimethylsilyl, Dimethylthexylsilyl, tert.Butyldiphenylsilyl, Trimethylsilylethoxycarbonyl, Benzyl, Triphenylmethyl (Trityl), Monomethoxytrityl (MMTr), Dimethyloxytrityl (DMTr), Benzyloxycarbonyl, 2-Nitrobenzyl, 4-Nitrobenzyl, 2-Nitrobenzyloxycarbonyl, 4-Nitrobenzyloxycarbonyl, tert.Butyloxycarbonyl, 4-Methoxybenzyl, 4-Methoxybenzyloxycarbonyl, Formyl, Acetyl, Trichloracetyl, 2,2,2-Trichlorethoxycarbonyl, 2,4-Dimethoxybenzyl, 2,4-Dimethoxybenzyloxycarbonyl, Methoxymethyl, Methylthiomethyl, Methoxyethoxymethyl, [2-(Trimethylsilyl)ethoxy]-methyl, 2-(Methylthiomethoxy)ethoxycarbonyl, Tetrahydropyranyl, Benzoyl, N-Succinimid, 4-Methylbenzoyl, 4-Nitrobenzoyl, 4-Fluorbenzoyl, 4-Chlorbenzoyl oder 4-Methoxybenzoyl. Bevorzugt ist tertiär Butyldimethylsilyl.

Die Abspaltung der Hydroxyschutzgruppe erfolgt in an sich bekannter Weise z.B. durch Säure, Base oder durch Zugabe von Tetrabutylammoniumfluorid oder erfolgt bei der Hydrolyse der Carbonsäure.

Als Lösemittel für die Verfahren eignen sich übliche organische Lösemittel, die sich unter den Reaktionsbedingungen nicht verändern. Hierzu gehören Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt sind für das Verfahren [A] (II) + (III) -> (IV) Diethylether, Tetrahydrofuran und Dimethylformamid. Besonders bevorzugt ist Dimethylformamid.

Als Basen für das erfindungsgemäße Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat, Kaliumcarbonat oder Cäsiumcarbonat, Erdalkalicarbonate wie Calciumcarbonat, oder Alkali- oder Erdalkalialkoholate wie Natrium- oder Kaliummethanolat, Natriumoder Kaliumethanolat oder Kalium-tertbutylat, oder organische Amine (Trialkyl(C₁-C₆)amine) wie Triethylamin, oder Heterocyclen wie 1,4-Diazabicyclo[2.2.2]octan (DABCO), 1,8-Diazabicyclo[5.4.0]undec-7-en (DBU), Pyridin, Diaminopyridin, Methylpiperidin oder Morpholin. Es ist auch möglich, als Basen Alkalimetalle wie Natrium oder deren Hydride wie Natriumhydrid einzusetzen. Bevorzugt sind Natriumhydrid, Kaliumcarbonat, Cäsiumcarbonat, Triethylamin, Trimethylamin, Pyridin, Kalium-tert.butylat, DBU oder DABCO. Ganz besonders bevorzugt ist für den Schritt [A] (II) + (III) -> (IV) ist Natriumhydrid.

Im allgemeinen setzt man die Base in einer Menge von 0,05 Mol bis 10 Mol, bevorzugt von 1 Mol bis 2 Mol bezogen auf 1 Mol der Verbindung der Formel (II) ein.

Das erfindungsgemäße Verfahren (II) + (III) -> (IV) wird im allgemeinen in einem Temperaturbereich von -20°C bis +60°C, bevorzugt von 0°C bis +60°C, durchgeführt.

Das erfindungsgemäße Verfahren (II) + (III) -> (IV) wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Hydrolyse der Carbonsäureester erfolgt nach üblichen Methoden, indem man die Ester in inerten Lösemitteln mit üblichen Basen behandelt, wobei die zunächst entstehenden Salze durch Behandeln mit Säure in die freien Carbonsäuren überführt werden oder mit Säure im Falle der t-Butylester.

Als Basen eignen sich für die Hydrolyse die üblichen anorganischen Basen. Hierzu gehören bevorzugt Alkalihydroxide oder Erdalkalihydroxide wie beispielsweise Natriumhydroxid, Lithiumhydroxid, Kaliumhydroxid oder Bariumhydroxid, oder Alkalicarbonate wie Natrium- oder Kaliumcarbonat oder Natriumhydrogencarbonat. Besonders bevorzugt werden Natriumhydroxid oder Lithiumhydroxid eingesetzt.

Als Säuren eignen sich im allgemeinen Trifluoressigsäure, Schwefelsäure, Chlorwasserstoff, Bromwasserstoff und Essigsäure oder deren Gemisch ggf. unter Zusatz von Wasser. Bevorzugt sind Chlorwasserstoff oder Trifluoressigsäure im Falle der Tertiär-Butylester und Salzsäure im Falle der Methylester.

Als Lösemittel eignen sich für die Hydrolyse Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, Dimethylformamid, Dichlormethan oder Dimethylsulfoxid. Ebenso ist es möglich, Gemische der genannten Lösemittel einzusetzen. Bevorzugt sind Wasser/Tetrahydrofuran, und im Falle der Umsetzung mit Trifluoressigsäure, Dichlormethan sowie im Falle von Chlorwasserstoff Tetrahydrofuran, Diethylether oder Wasser.

Die Hydrolyse wird im allgemeinen in einem Temperaturbereich von 0°C bis +100°C durchgeführt.

Im allgemeinen wird die Hydrolyse bei Normaldruck durchgeführt. Es ist aber auch möglich, bei Unterdruck oder bei Überdruck zu arbeiten (z.B. von 0,5 bis 5 bar).

Bei der Durchführung der Hydrolysen wird die Base oder die Säure im allgemeinen in einer Menge von 1 bis 100 mol, bevorzugt von 1,5 bis 40 mol bezogen auf 1 mol des Esters eingesetzt.

Die Aktivierung der Carbonsäuren (V) erfolgt gewöhnlich durch deren Umwandlung in die entsprechenden Säurehalogenide, bevorzugt Säurechloride, oder eine Voraktivierung mit einem üblichen Kondensationsmittel, was in situ oder durch Isolation des aktivierten Carbonsäurederivats geschehen kann. Die Säurehalogenide können nach üblichen Methoden hergestellt werden. Beispielsweise seien die Verwendung von Oxalylchlorid oder Thionylchlorid genannt.

Als Hilfsstoffe für die Amidbildungen werden bevorzugt Kondensationsmittel eingesetzt. Bevorzugt werden hier die üblichen Kondensationsmittel wie Carbodiimide z.B. N,N'-Diethyl-, N,N'-Dipropyl-, N,N'-Diisopropyl-, N,N'-Dicyclohexylcarbodiimid, N-(3-Dimethylaminoisopropyl)-N'-ethylcarbodiimid-Hydrochlorid (EDC), oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri-(dimethylamino)phosphoniumhexafluorophosphat, und als Basen Alkalicarbonate z.B. Natrium- oder Kaliumcarbonat, oder -hydrogencarbonat, oder organische Basen wie Trialkylamine z.B. Triethylamin, N-Ethylmorpholin, N-Methylpiperidin oder Diisopropylethylamin eingesetzt. Besonders bevorzugt ist die Kombination von EDC, N-Methylmorpholin und 1-Hydroxybenztriazol. Als Lösungsmittel für die Amidbildung sind Dichlormethan und DMF bevorzugt.

Die Verbindungen der allgemeinen Formeln (II), (IIIa), (VI) und (VIII) sind bekannt oder nach üblichen Methoden herstellbar (vgl. EP-A-0 725 061, EP-A-0 725 064).

Die Verbindungen der allgemeinen Formel (III) sind größtenteils neu und können hergestellt werden, indem man, im Fall, daß R¹¹ nicht für einen direkt über N-gebundenen Heterocyclus steht, Verbindungen der allgemeinen Formel (XI) 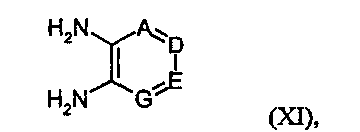 in welcher
A, D, E, G und L¹ die oben angegebene Bedeutung haben,
mit Verbindungen der allgemeinen Formel (XII)

R¹¹-CO₂H (XII)

in welcher
R¹¹ die oben angegebene Bedeutung hat,
unter Entfernung des Reaktionswassers, gegebenenfalls in Anwesenheit einer Säure, vorzugsweise mit PPA, HCl und p-TsOH, umsetzt (vgl. hierzu auch *J. Org. Chem.* **1941,** *6*, 25 ff und *Bull. Soc. Chim. Fr.* **1991**, *128*, 255-259)
und im Fall, daß R¹¹ für einen der oben unter R² aufgeführten Reste steht, der gegebenenfalls auch eine Schutzgruppe trägt, Verbindungen der allgemeinen Formel (XI) zunächst durch Umsetzung mit Verbindungen der allgemeinen Formel (XIII)

HO-R¹⁴-CO₂H (XIII)

in welcher
R¹⁴ für (C₁-C₈)-Alkandiyl steht,
in die Verbindungen der allgemeinen Formel (XIV) 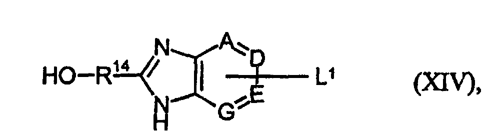 in welcher
A, B, D, G, R¹⁴ und L¹ die oben angegebene Bedeutung haben,
in inerten Lösemitteln überführt,
anschließend die Hydroxygruppe durch Halogen, Mesylat oder Tosylat substituiert und somit die Verbindungen der allgemeinen Formel (XV) 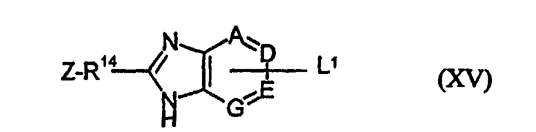 in welcher
R¹⁴, A, D, E, G und L¹ die oben angegebene Bedeutung haben
   und
Z für Halogen, Mesylat oder Tosylat steht,
herstellt, und diese mit Aminen der allgemeinen Formel (XVI)

R⁸R⁹NH (XVI)

in welcher
R⁸ und R⁹ die oben angegebene Bedeutung haben,
umsetzt (vgl. hierzu auch *J. Am. Chem. Soc.* **1948**, *70*, 3406; *J. Heterocycl. Chem.* **1969**, 759-60).

Als Lösemittel für das Verfahren eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt sind Dichlormethan, Tetrahydrofuran und Dimethylformamid.

Als Basen für das erfindungsgemäße Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat, Kaliumcarbonat oder Cäsiumcarbonat, Erdalkalicarbonate wie Calciumcarbonat, oder Alkali- oder Erdalkalialkoholate wie Natrium- oder Kaliummethanolat, Natriumoder Kaliumethanolat oder Kalium-tert.butylat, oder organische Amine (Trialkyl(C₁-C₆)amine) wie Triethylamin, oder Heterocyclen wie 1,4-Diazabicyclo[2.2.2]octan (DABCO), 1,8-Diazabicyclo[5.4.0]undec-7-en (DBU), Pyridin, Diaminopyridin, Methylpiperidin oder Morpholin. Es ist auch möglich, als Basen Alkalimetalle wie Natrium oder deren Hydride wie Natriumhydrid einzusetzen. Bevorzugt sind Natriumhydrid, Kaliumcarbonat, Triethylamin, Trimethylamin, Pyridin, Kaliumtert.butylat, DBU oder DABCO.

Im allgemeinen setzt man die Base in einer Menge von 0,05 Mol bis 10 Mol, bevorzugt von 1 Mol bis 2 Mol bezogen auf 1 Mol der Verbindung der Formel (XV) ein.

Das erfindungsgemäße Verfahren wird im allgemeinen in einem Temperaturbereich von -50°C bis +100°C, bevorzugt von -30°C bis +60°C, durchgeführt.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Verbindungen der allgemeinen Formeln (XI), (XII), (XIII) und (XVI) sind an sich bekannt oder nach üblichen Methoden herstellbar.

Die Verbindungen der allgemeinen Formeln (XIV) und (XV) sind teilweise neu und können beispielsweise wie oben beschrieben hergestellt werden.

Die Verbindungen der allgemeinen Formel (IV), (V), (VII), (IX) und (X) und deren Salze sind neu und können wie oben beschrieben hergestellt werden.

Überraschenderweise zeigen die erfindungsgemäßen Verbindungen der allgemeinen Formel (I) und ihre Analoga ein nicht vorhersehbares, wertvolles pharmakologisches Wirkspektrum kombiniert mit einer verbesserten Wasserlöslichkeit.

Es wurde gefunden, daß die erfindungsgemäßen Verbindungen die Adenosinaufnahme hemmen.

Sie können in oraler oder intravenöser Applikation zur Vorbeugung und Behandlung von cerebraler Ischämie, Hirnschlag, Reperfusionsschäden, Hirntrauma, Ödemen, Krämpfen, Epilepsie, Atemstillstand, Herzstillstand, Reye-Syndrom, cerebraler Thrombose, Embolie, Tumoren, Blutungen, Enzephalomyelitis, Hydroenzephalitis, Rückenmarksverletzungen, post-operative Hirnschäden, Verletzungen der Retina oder des optischen Nervs nach Glaukom, Ischämie, Hypoxie, Ödem oder Trauma sowie in der Behandlung von Schizophrenie, Schlafstörungen und Schmerz eingesetzt werden.

Aufgrund ihrer verbesserten Wasserlöslichkeit eignen sich die erfindungsgemäßen Verbindungen besonders gut zur intravenösen Applikation.

### Testsysteme

### 1. Bestimmung der Löslichkeit

Zur Löslichkeitsbestimmung wurde eine Fällungsmethode herangezogen: 10 mg der Testsubstanz werden in 50µl DMSO vollständig gelöst (Stammlösung). Von dieser Lösung gibt man 20µl in 2000µl physiologische Kochsalzlösung. Diese Lösung wiederum wird zur Equilibrierung bei 25°C im Thermomixer Comfort (Fa. Eppendorf) bei 1400 rpm 24 Stunden geschüttelt.

Die ausgefallenen Teile der Testsubstanz werden mit der Biofuge 15 Fa. Heraeus 5 min bei 14000 rpm abzentrifugiert. 1300 µl des Überstandes werden erneut mit der Microfuge Fa. Beckmann bei 45000 rpm = 125000g zentrifugiert.

10 µl dieses Zentrifugationsüberstandes werden nun mit 1000µl DMSO verdünnt und diese Lösung an der HPLC gemessen. (Fa. Hewlett Packard 1090, Methode: Gradient von 100% PBS-Puffer pH=4 innerhalb von 15 min auf 10% Puffer/90% Acetonitril, Säule: RP18; PBS-Puffer pH=4 steht dabei für eine physiologische Kochsalzlösung, die mit Phosphatpuffer auf pH=4 eingestellt wurde).

Die gemessene Peakfläche der HPLC-Messung wird mit einer Eichgerade auf die Substanzkonzentration umgerechnet. Für die Eichgerade werden 20µl der Stammlösung sukzessiv mit DMSO so verdünnt, daß 5 Konzentrationen von 2.5 mg/l bis 2000mg/l entstehen. Diese Lösungen werden ebenfalls an der HPLC gemessen (Methode s. o.) und die Peakflächen gegen die Konzentrationen aufgetragen.

Die nach dieser Methode bestimmte Löslichkeit der Beispiele 3 und 5 beträgt 176 bzw. 16 mg/l.

### 2. Bindung der erfindungsgemäßen Verbindungen an ein Adenosin-Transport-Protein aus Kalbs-Kortex

Die Fähigkeit von Substanzen das Adenosinaufnahme-System zu beeinflussen wird erstens durch die Bestimmung der Bindungsaffinität ausgewählter Substanzen zu einem Adenosin-Transportprotein des ZNS und zweitens durch die Bestimmung der hemmenden Wirkung der Substanzen auf die funktionelle Adenosinaufnahme untersucht.

Für den Bindungstest wird eine Membranpräparation des cerebralen Kortex vom Kalb verwendet, welcher den relevanten Adenosintransporter exprimiert. Die Bindungsaffinität (Kᵢ-Wert) wird bestimmt, indem die Verdrängung eines spezifischen, radioaktiv markierten Liganden [Nitrobenzylthioinosin (NBTI)] von der betreffenden Bindungsstelle durch Test-Substanzen gemessen wird. Es handelt sich um die, für den eigentlichen Transportprozess relevante, Bindungsstelle auf dem Transportprotein. Die Bindung von Testsubstanzen in diesem Versuch führt daher zu einer meßbaren Freisetzung von gebundenem, radioaktiven NBTI, welche die Bestimmung des Kᵢ-Wertes ermöglicht (*J. Neurochemistry* **1982**, *39*, 184-191).

Die Beispiele 3 und 5 inhibieren die Bindung von NBTI mit jeweils Kᵢ= 2 nM.

### 3. Hemmung der Adenosinaufnahme in Kalbs-Kortex Synaptosomen durch erfindungsgemäße Verbindungen

Für den funktionellen Adenosinaufnahme-Test wird eine Synaptosomen-Präparation aus dem cerebralen Kortex vom Kalb verwendet, die den betreffenden Adenosin-Transporter exprimiert. Synaptosomen sind zellfreie, funktionell aktive Vesikel, die aus Kortexgewebe unter Einsatz von Scherkräften gewonnen werden und noch die Eigenschaften eines intakten synaptischen Endknöpfchens besitzten. Die Hemm-Aktivität (IC₅₀ -Wert) wird bestimmt, indem die Hemmung der Aufnahme des spezifischen, radioaktiv markierten "Substrats" Adenosin in die Synaptosomen gemessen wird (*J. Neurochemistry* **1990**, *55*, 541-550).
Die Beispiele 3 und 5 hemmen die Adenosin-Aufnahme in Synaptosomen mit IC₅₀= 8 nM bzw. 14 nM.

Die neuroprotektive Wirksamkeit der erfindungsgemäßen Verbindungen wurde im Tier-Modell der transienten Okklusion der Mittleren Cerebral-Arterie (tMCA-O) und des Subduralen Hämatoms (SDH) bestimmt.

### 4. tMCA-O

Dieses Nagetiermodell (Ratte) imitiert die Pathophysiologie und cerebrale Pathologie des Schlaganfalls bzw. Durchblutungsstillstands (Embolisation, Thrombose, Vasospasmus, Herzstillstand, rapide u. dramatische Blutdrucksenkung, hoher Blutverlust, usw.) mit nachfolgender Rezirkulation beim Menschen (Modifiziert nach: *J. Cereb. Blood Flow Metab.* **1997**, *17*, 1066-1073).

Unter Allgemeinnarkose (Inhalationsnarkose mit Isofluran) werden die Haare im unteren vorderen Halsbereich abrasiert, bei Rückenlage der Kopf fixiert, die Haut desinfiziert und im Halsbereich mittig entlang der Trachea geöffnet. Die rechte seitliche Halsmuskulatur wird längs stumpf durchtrennt und zusammen mit der Haut auf die Seite gezogen (Wundhaken), so daß die Arteria carotis communis deutlich sichtbar ist. Die Arteria carotis communis wird in Richtung Kopf freipräpariert, bis sie sich in die Arteria carotis interna und Arteria carotis externa teilt. Mit Hilfe von chirurgischem Nahtmaterial werden die Arteria carotis communis (nahe des Brustbereiches) und Arteria carotis externa abgebunden. Die Arteria carotis interna wird mit einer Mikroklemme temporär verschlossen. Nach Öffnung der Arteria carotis communis wird ein Nylon Monofilament mit abgerundeter Spitze und einem 1 cm langen Siliconzylinder durch die Arteria carotis communis dann nach Öffnen der Mikroklemme über die Arteria carotis interna vorgeschoben um den Abgang der Mittleren Cerebralen Arterie (Arteria cerebri media) zu verschließen. Mit zwei temporären Fadenschlingen wird das Filament in der Arteria carotis interna fixiert. Nach einer Stunde wird das Filament herausgezogen, und die Arteria carotis interna sowie Arteria carotis communis oberhalb der Öffnung abgebunden. Die Blutzufuhr erfolgt durch das contralaterale Gefäßsystem.

Die Substanzapplikation beginnt direkt mit dem Beginn der Reperfusion. Die Operationswunde wird chirurgisch versorgt. Während der Operation und der Substanzapplikation (Infusion) wird die Körpertemperatur durch eine Wärmeplatte konstant gehalten.

Nach 2 Tagen post-operativer Überlebenszeit wird das Volumen des cerebralen Infarktes mit Hilfe eines computerunterstützten Bildanalysesystems an vorgefertigten histologischen Schnittserien des Gehirns bestimmt. Die Infarktgröße wird nach Cortex, Striatum, Hippocampus und sonstigen Hirngebieten differenziert ausgewertet.

Bei einer Dosis von 0,001 mg/(kg x h) (i.v. Infusion) reduzieren die Beispiele 3 und 5 das Infarktvolumen um 81 bzw. 91% im Vergleich zu Kontrolltieren.

### 5. Subdurales Hämatom bei der Ratte (SDH)

Dieses Nagetiermodell (Ratte) imitiert die Pathophysiologie und cerebrale Pathologie des stumpfen Schädel-Hirn-Traumas mit subduraler Blutung und Entwicklung eines subduralen Hämatoms beim Menschen (*Neurosurgery* **1990**, *27*, 433-439).

Unter Anästhesie wird den Tieren einseitig subdural Eigenblut injiziert. Unter dem Hämatom bildet sich ein Infarkt. Die Substanzapplikation erfolgt nach unterschiedlichen zeitlichen Schemata und über unterschiedliche Applikationswege (i.v., i.p.). Die Bestimmung der Infarktgröße erfolgt wie beim Modell der transienten focalen Ischämie bei der Ratte (tMCA-O) beschrieben.

Bei einer Dosis von 0,001 mg/(kg x h) (i.v. Infusion) reduzieren die Beispiele 3 und 5 das Infarktvolumen um 30 bzw. 45% im Vergleich zu Kontrolltieren.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,0001 bis 90 Gew.-%, bevorzugt 0,0001 bis 1,0 Gew.-%, der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral, transdermal oder parenteral, insbesondere perlingual oder intravenös.

Im allgemeinen hat es sich als vorteilhaft erwiesen, bei intravenöser Applikation Mengen von etwa 0,00001 bis 10 mg/kg, vorzugsweise etwa 0,0001 bis 1 mg/kg Körpergewicht zur Erzielung wirksamer Ergebnisse zu verabreichen.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

### Abkürzungen

- DMF:: N,N-Dimethylformamid
- DMSO:: Dimethylsulfoxid
- PPA:: Polyphosphorsäure
- TFA:: Trifluoressigsäure
- THF:: Tetrahydrofuran

### Ausgangsverbindungen

### Beispiel 1A

**(1*R*, 2*R*)-2-(4-Methyl-phenyl)-cyclohexan-1-carbonsäure**

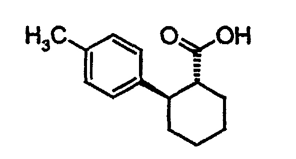

Racemische (1R*,2R*)-2-(4-Methyl-phenyl)-cyclohexan-1-carbonsäure wurde analog dem in US-A-5,395,840, Spalte16, beschriebenen Verfahren hergestellt. Das erhaltene racemische Material wurde nach folgender Vorschrift in die Enantiomeren getrennt:

Die racemische Säure (415 g; 1,9 mol) und Triethylamin (96,2 g; 0,95 mol; 131,8 ml) wurden in einem Gemisch aus THF (2,7 l) und Wasser (5,3 l) suspendiert. S-(-)-Phenethylamin (115,2 g; 0,95 mol) wurde bei 60 °C tropfenweise hinzugefügt, wobei ein Niederschlag ausfiel. Das Gemisch wurde 2 h bei 60 °C gerührt und anschließend mit einem Eisbad gekühlt. Dieser Niederschlag wurde abgesaugt und enthielt überwiegend das Phenethylaminsalz des (1S,2S)-Enantiomers. Das Filtrat wurde mit konz. HCl angesäuert und zweimal mit Dichlormethan extrahiert. Die vereinigten Extrakte wurden über Natriumsulfat getrocknet und eingeengt. Ausb.: 202,4 g (28%) eines mit dem (1*R*, 2*R*)-Isomer angereicherten Enantiomerengemisches.

Dieses Gemisch wurde wie oben beschrieben mit R-(+)-Phenethylamin behandelt, um das gewünschte Enantiomer als Salz zu fällen. Die farblosen Kristalle wurden abgesaugt und aus Acetonitril/Methanol (6:1) umkristallisiert. Die Röntgenstrukturanalyse dieser Kristalle ergab die (1*R*, 2*R*)-Konfiguration. Ausb. 136,9 g (46%). Nach der Aufarbeitung (s.o.) wurden 89 g (1*R*, 2*R*)-2-(4-Methylphenyl)-cyclohexan-1-carbonsäure erhalten.

### Beispiel 2A

**(1*R*,2*R*)-2-(4-Bromomethyl-phenyl)-cyclohexan-1-carbonsäure-tert-butylester:**

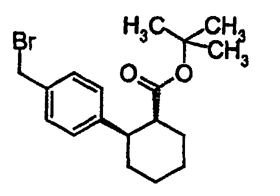

Das Zwischenprodukt wurde in Analogie zur Vorschrift für das Racemat (US-A-5,395,840, Spalte 17) hergestellt. Zur Aufreinigung wurde das erhaltene Gemisch mit Diethylether verrührt.

### Beispiel 3A

**2-(2-Phthalimidylethyl)-benzimidazol**

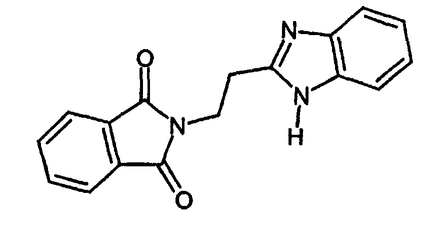

2-Aminoethylbenzimidazol-Dihydrochlorid (*Bull. Soc. Chim. Fr*. **1991**, *128*, 255-259; 2,34 g, 10 mmol), Phthalsäureanhydrid (1,63 g, 11 mmol) und Triethylamin (2,79 ml, 20 mmol) wurden über Nacht in Chloroform (25 ml) zum Rückfluß erhitzt, danach auf Raumtemperatur abgekühlt, mit Essigester verdünnt und abfiltriert. Das Filtrat wurde mit gesättigter Natriumcarbonatlösung, Puffer (pH=7) und gesättigter Kochsalzlösung gewaschen und über Natriumsulfat getrocknet. Chromatographie (Dichlormethan:Methanol 10:1, R_{f}=0,4) lieferte 2,08 g 2-(2-Phthalimidylethyl)-benzimidazol (71,4% der Theorie) als farblosen Schaum. MS (DCI, NH₃)=292 (M+H⁺). ¹H-NMR (DMSO-d₆): 3,15 (2 H, t); 4,0 (2 H, t); 7,05-7,2 (2 H, m); 7,4-7,5 (2 H, m); 7,8-7,9 (4 H, m); 12,4 (1 H, br s).

Im weiteren Verlauf der Synthese wird nach den allgemeinen Arbeitsvorschriften A, B und C wie unten angegeben verfahren und in einem letzten Schritt wie weiter unten beschrieben die Phthalimidgruppe abgespalten.

### Beispiel 4A

**2-(2-Hydroxyethoxymethyl)-pyrido[2,3-*d*]imidazol**

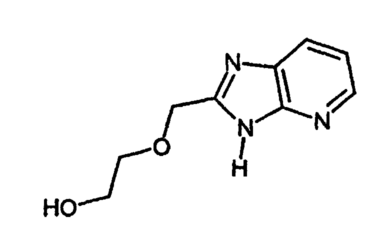

1,4-Dioxan-2-on (6,13 g, 60 mmol) und 2,3-Diaminopyridin (5,46 g, 50 mmol) wurden am Wasserabscheider während 10 h in Mesitylen (100 ml) auf Rückfluss erhitzt. Nach dem Abkühlen wurde Mesitylen abdekantiert und der Rückstand durch Chromatographie über Kieselgel (Dichlormethan:Methanol 9:1) gereinigt (Ausbeute: 8,47 g, 87% der Theorie).

MS(DCI)=194 (M+H, 100%); ¹H-NMR (DMSO-d₆): 3,78 (2 H, m); 3,89 (2H, m); 4,91 (2 H, s); 5,3 (1 H, s); 7,18 (1 H, dd); 7,95 (1 H, d); 8,43 (1 H, dd); 12,7 (1 H, br s).

### Beispiel 5A

**2-[2-(*tert*-Butyldimethylsilyloxy)ethoxymethyl]-pyrido[2,3-*d*]imidazol**

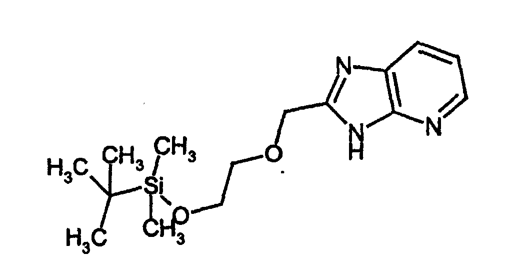

8,4 g (43,48 mmol) 2-(2-Hydroxyethoxymethyl)-(pyrido-[2,3-*d*]-*1H*-imidazol) und 4,84 g (47,82 mmol) Triethylamin wurden in 120 ml DMF gelöst und mit 7,21 g (47,8 mmol) TBDMS-Chlorid versetzt, wobei sich das Gemisch auf ca. 40°C erwärmte. Nach dem noch 2 h bei Raumtemp. gerührt wurde, goß man das Gemisch auf Wasser, wobei das Produkt kristallin anfiel. Man saugte es ab, wusch mit wenig Wasser nach und trocknete im Hochvakuum. ¹H-NMR (DMSO-d₆): 0,02 (6H, s); 0,83, (9H, s); 3,52 (2 H, t); 3,75 (2H, t); 4,73 (2 H, s); 7,18 (1 H, dd); 7,90 (1 H, dd); 8,43 (1 H, dd); 12,9 (1 H, br s).

### Beispiel 6A

**2-*tert*-Butyldimethylsilyloxymethyl-benzimidazol:**

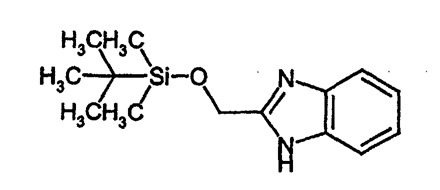

Zu einer Lösung aus 2-Hydroxymethylbenzimidazol (1,48 g, 9,95 mmol) in DMF (30 ml) wurde bei Raumtemperatur Triethylamin (2,27 ml, 16,3 mmol) und TBDMS-Chlorid (1,65 g, 10,95 mmol) gegeben. Nach 3,5 h wurde die Reaktion durch Zugabe von Wasser abgebrochen, mit Diethylether extrahiert, und die organische Phase über Natriumsulfat getrocknet. Chromatographie (Kieselgel, Cyclohexan:Essigester 2:1, R_{*f*}=0,35) lieferte 2,52 g 2-*tert*-Butyldimethylsilyloxymethyl-benzimidazol (97% der Theorie) als bräunliches Pulver. MS (DCI, NH₃)=263 (M+H⁺). ¹H-NMR (DMSO-d₆): 0,00 (6 H, s); 0,80 (9 H, s); 4,75 (2 H, s); 7,0-7,1 (2 H, m); 7,4-7,5 (2 H, m); 12,15 (1 H, br s).

### Beispiel 7A

**2-(2-Hydroxyethoxymethyl)-benzimidazol:**

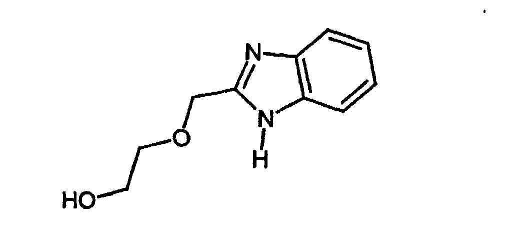

1,4-Dioxan-2-on (2,04 g, 20 mmol) und 1,2-Diaminobenzol (2,16 g, 20 mmol) wurden am Wasserabscheider während 10 h in Mesitylen (150 ml) auf Rückfluss erhitzt. Anschliessend wurden die beim Abkühlen entstandenen Kristalle abgenutscht (2,94 g, 77% der Theorie). R_{*f*} (Dichlormethan:Methanol 10:1) =0,45, MS (EI)=192 (M⁺, 20%), 148 (20%), 147 (40%), 132 (100%). ¹H-NMR (DMSO-d₆): 3,6 (4 H, s); 4,65 (1 H, s); 4,7 (2 H, s); 7,1-7,2 (2 H, m); 7,45 (1 H, d); 7,55 (1 H, d); 12,4 (1 H, br s).

### Allgemeine Vorschrift zur Alkylierung [A]:

In einem typischen Ansatz wurde bei 0°C Natriumhydrid (6,3 mmol) zu einer Lösung des Imidazols der allgemeinen Formel (III) (6 mmol) in trockenem DMF (30 ml) gegeben. Nach 30 min bei Raumtemperatur und 30 min bei 40°C wurde bei 0°C die Verbindung der allgemeinen Formel (II) (6,3 mmol) zugegeben und das Reaktionsgemisch über Nacht bei Raumtemperatur gerührt. Die Reaktion wurde daraufhin durch Zugabe von Wasser abgebrochen, mit Diethylether extrahiert, und die organische Phase anschliessend über Natriumsulfat getrocknet. Nach Chromatographie (Kieselgel, Cyclohexan:Essigester) wurde Produkt in 60-70% Ausbeute erhalten.

### Allgemeine Vorschrift zur Esterspaltung [B]:

In einem typischen Ansatz wurde bei Raumtemperatur Trifluoressigsäure (5 ml) zu einer Lösung aus Ester der allgemeinen Formel (IV) (T= *tert*-Bu; 1,5 mmol) in Dichlormethan (5 ml) gegeben. Nach 2 h wurde auf 0°C gekühlt, mit wässriger Natriumhydroxidlösung (ca. 30 ml, 2 M) auf pH=2 gestellt und mit Dichlormethan extrahiert. Trocknen der organischen Phase über Natriumsulfat lieferte nach Einengen die Verbindung der allgemeinen Formel (V).

### Allgemeine Vorschrift zur Amidbildung [C]:

Eine Suspension aus Säure (V) (4 mmol), *(S)*-Phenylglycinamid-Hydrochlorid (4,2 mmol), 1-Hydroxybenzotriazol (4,4 mmol), EDC-Hydrochlorid (4,8 mmol) und Triethylamin (12 mmol) in Dichlormethan (40 ml) wurde während 24-48 h bei Raumtemperatur gerührt. Nach Zugabe von Wasser wurde mit Dichlormethan (z.T. mit Methanol) extrahiert, die organische Phase über Natriumsulfat (oder Magnesiumsulfat) getrocknet und chromatographiert (Kieselgel, Dichlormethan:Methanol). Es wurde gewünschtes Produkt in 60-80% Ausbeute erhalten.

Analog kann nach Vorschrift C statt Phenylglycinamid Phenylglycinol eingesetzt werden.

### Herstellungsbeispiele

### Beispiel 1

**(*S*)-N-{(1*R**, 2*R**)-{4-[2-(2-Aminoethyl-benzimidazol-1-yl)methyl]phenyl}-cyclohex-2-yl-carbonyl}-phenylglycinamid**

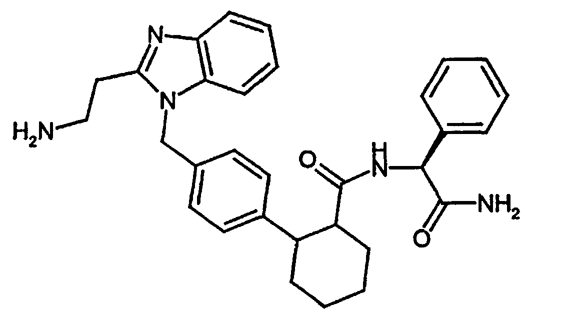

Zu einer Suspension aus *(2S)*-N-[*(2R*)*-(4-{2-(2-Phthaloylaminoethyl)-benzimidazol-1-yl-methyl}-phenyl)-cyclohexyl-*(1R*)*-carbonyl]-phenylglycinamid (hergestellt nach den allgemeinen Vorschriften [A - C] aus der Verbindung des Beispiels 3A und dem Racemat von Beispiel 2A gemäß US-A-5,395,840, Example IV; 500 mg, 0,78 mmol, Diastereomerengemisch) in Ethanol (25 ml) wurde Hydrazinhydrat (0,38 ml, 7,82 mmol) gegeben. Das Gemisch wurde über Nacht bei Raumtemperatur gerührt, dann mit Salzsäure (1 M) auf pH=2 gestellt und eingeengt. Extraktion zwischen 10% wässriger Natriumbicarbonatlösung und Dichlormethan, Trocknen der organischen Phase über Natriumsulfat und Chromatograpie (Kieselgel, Dichlormethan: Methanol:konz. wässriger Ammoniak 100:13:1,3, Rf(10:1:0,2)=0,1) lieferte die Titelverbindung (292 mg, 72%, Diastereomerengemisch) als gelbliches Pulver. MS (DCI, NH₃)=510 (M+H⁺). ¹H-NMR (DMSO-d₆): 1,2-1,5 (4 H, m); ); 1,6-1,9 (4 H, m); 2,0 (2 H, br s); 2,6-3,0 (6 H, m); 5,1-5,2 (A:1 H, d; B:1 H, d); 5,4-5,5 (A:2 H, s; B:2 H, s); 6,85-7,0 (4 H, m); 7,1-7,3 (7 H, m); 7,4-7,5 (1 H, m); 7,55-7,65 (4 H, m); 8,05-8,15 (A:1 H, d; B:1 H, d).

### Beispiel 2

**(*S*)-N-{(1*R*, 2*R*)-{4-{[2-(2-Aminoethyl)-benzimidazol-1-yl]methyl}phenyl}-cyclohex-1-yl-carbonyl}phenylglycinamid dihydrochlorid**

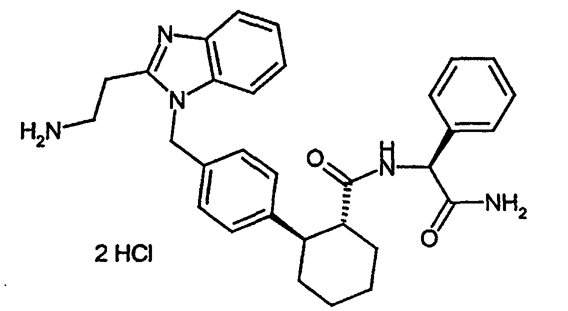

Chromatographische Trennung des Eduktes von Beispiel 1 (Kieselgel, Methylenchlorid: Methanol) lieferte diastereomerenreines *(S)*-(N)-{(1*R*, 2*R*)-2-{4{2-[2(Phthaloyl-amino)-ethyl]-benzimidazol-1-yl}methyl}-phenyl}-cyclohex-1-yl-carbonyl}phenylglycinamid, das analog Beispiel 1 entschützt wurde und dann in einer möglichst geringen Menge Dichlormethan vollständig in Lösung gebracht und mit etwa 2 Äquivalenten 1M-HCl in Diethylether behandelt und eingeengt wurde.
Gef.: C 64,21 H 6,58
Ber.: C 63,91 H 6,49

### Beispiel 3

**(*S*)-N-{{(1*R*, 2*R*)-{4-{2-[2-(Morpholin-4-yl-methyl)-1*H*-pyrido[2,3-d]imidazol-1-yl]methyl}-phenyl}-cyclohex-1-yl}carbonyl}-phenylglycinamid** 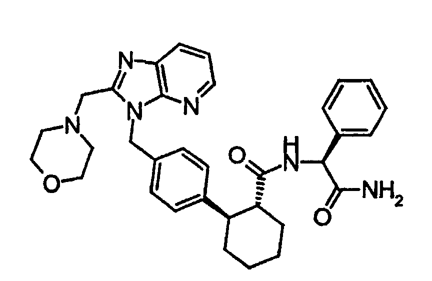
a) 2-Hydroxymethyl-1*H*-pyrido[2,3-*d*]imidazol 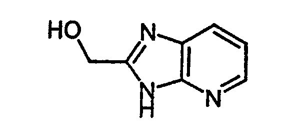
   2,3-Diaminopyridin (54,6 g; 0,5 mol) und Glykolsäure (38 g; 0,5 mol) wurden in 700 mL Mesitylen am Wasserabscheider unter Rückfluß gekocht, bis sich die berechnete Menge Wasser abgeschieden hatte. Anschließend wurde das Gemisch auf Raumtemperatur gekühlt, der entstandene Niederschlag abgesaugt und 15 min in 800 mL Wasser unter Zusatz von Aktivkohle gekocht. Nachdem die Suspension heiß filtriert und wieder auf Raumtemperatur abgekühlt wurde, fielen farblose Kristalle aus, die abgesaugt und getrocknet wurden. Ausbeute: 56,4 g (75%).
b) 2-Chlormethyl-1*H*-pyrido[2,3-*d*]imidazol Hydrochlorid: 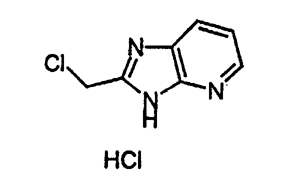
   Die Verbindung aus Beispiel 3a (14,9 g; 100 mmol) wurde in 25 mL Ethanol suspendiert und ein trockener HCl-Strom bis zur Sättigung eingeleitet. Das erhaltene Hydrochlorid wurde abgesaugt und im Vakuum getrocknet. Ausb. 18,1 g (100%). Dieses wurde in 100 mL Chloroform suspendiert und mit 35 mL Thionylchlorid versetzt. Anschließend wurde das Gemisch 24 h unter Rückfluß erhitzt, dann heiß filtriert und der Niederschlag mit Chloroform gewaschen und im Vakuum getrocknet. Ausb. 18,9 g (92%).
c) 2-(Morpholin-4-yl-methyl)-1*H*-pyrido[2,3-*d*]imidazol: 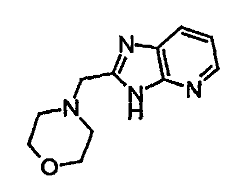
   Die Verbindung aus Beispiel 3b (13,7 g; 67 mmol) und Morpholin (28,6 g; 328 mmol) wurden 3 h unter Rückfluß gekocht. Das Gemisch wurde eingeengt und der Rückstand mit Natriumhydrogenkarbonatlösung aufgenommen. Diese Suspension wurde 15 min unter Zusatz von Aktivkohle gekocht und anschließend heiß filtriert. Nachdem das Gemisch eingeengt wurde, reinigte man das entstandene Produkt durch Säulenchromatographie (Kieselgel (70-230 mesh ASTM); Eluent: 100:30:1 Essigester/ Ethanol/ Triethylamin). Das Produkt kann aus Essigester/Hexan umkristallisiert werden.
d) (1*R*, 2*R*)-{4-{[2-(Morpholin-4-yl-methyl)-1*H*-pyrido[2,3-*d*]imidazol-1-yl]methyl}-phenyl}-cyclohexan-1-carbonsäure*-tert*-butylester 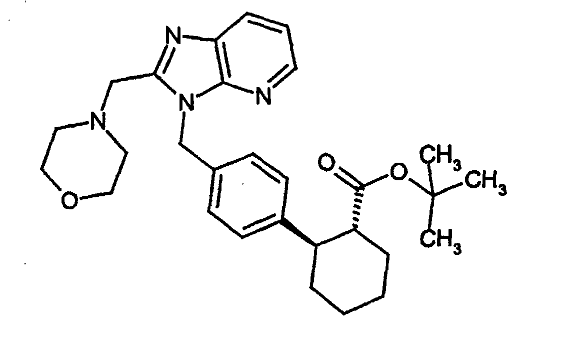
   Eine 60%ige Suspension von Natriumhydrid in Öl (2 g; 51,6 mmol) wurde unter Argon in 150 mL DMF suspendiert und die Verbindung aus Beispiel 3c (9,5 g; 43,5 mmol) hinzugefügt. Das Gemisch wurde 30 min auf 50 °C erwärmt, wobei sich ein Niederschlag bildete. Anschließend wurde auf Raumtemperatur gekühlt und die Verbindung aus Beipiel 2A (17,3 g; 44 mmol) hinzugefügt, wonach das Gemisch 20 h bei Raumtemp. gerührt wurde. Die entstandene klare Lösung wurde im Hochvakuum eingeengt und der Rückstand mit Dichlormethan/ Wasser aufgenommen. Die organische Phase wurde abgetrennt, über Natriumsulfat getrocknet und eingeengt. Der Rückstand wurde anschließend durch Säulenchromatographie gereinigt (Kieselgel (70-230 mesh ASTM); Eluent: 100:4 Dichlormethan/ Methanol) Ausb. 10 g (47%) eines braunen, viskosen Öls.
e) (1*R*, 2*R*)-2-{4-{[2-(Morpholin-4-yl-methyl)-1*H*-pyrido[2,3-*d*]imidazol-1-yl]methyl}phenyl}cyclohexan-1-carbonsäure 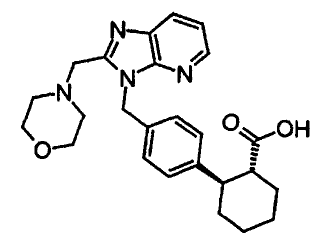
   Die Verbindung aus Beispiel 3d (10 g; 20,4 mmol), 120 mL Dichlormethan und 100 mL Trifluoressigsäure wurden 1 h bei Raumtemp. gerührt. Anschließend wurde das Gemisch unter Kühlung mit konz. Natronlauge neutralisiert, die org. Phase abgetrennt, getrocknet und eingeengt. Der Rückstand wurde durch Säulenchromatographie gereinigt. (Eluent: Dichlormethan/ Methanol 100:6) Ausb. 7,3 g (80%) eines farblosen amorphen Festkörpers.
f) (*S*)-N-{{(1*R*, 2*R*)-2-{4-{[2-(Morpholin-4-yl-methyl)-1*H*-pyrido[2,3-d]imidazol-1-yl]methyl}phenyl}-cyclohex-1-yl}carbonyl}-phenylglycinamid 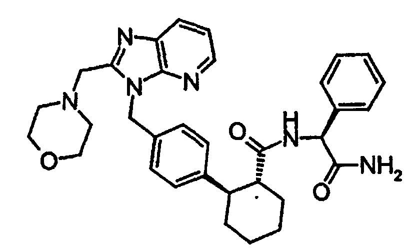
   Gemäß dem allgemeinen Verfahren [C] wurde die Verbindung von Beispiel 3e (1,4 g; 3,22 mmol) unter Zusatz einer Spatelspitze DMAP (4-Dimethylaminopyridin) umgesetzt. Zur Aufarbeitung wurde das Produkt mit Dichlormethan extrahiert und durch Säulenchromatographie gereinigt (Dichlormethan/ Methanol 100:6). Ausb. 1,7 g (93%) eines blaßgelblichen Pulvers.
   ¹H-NMR (300 MHz; CDCl₃) δ[ppm]: 1,25-1,5 (3H; br m), 1,62 (1H; dq), 1,8 (3H; m), 1,94 (1H; dd), 2,31 (1H; dt), 2,42 (4H, br m), 2,67 (1H; dt), 3,61 (6H; m), 5,21 (1H; d), 5,49 (1H, br s), 5,63 (2H; d+d), 5,72 (1H; br s), 6,41 (1H; d), 6,82 (2H; d), 6,92 (2H; d), 6,98 (2H; d), 7,13 (2H, t), 7,18 (1H; t), 7,23 (1H; dd), 8,03 (1H; d), 8,42 (1H; d)
   MS (DCI/NH₃)[m/z]: 567 (100, M+H)

### Beispiel 4

**(*S*)-N-{{(1*R*, 2*R*)-{4-{2-[2-(Morpholin-4-yl-methyl)-1*H*-pyrido[2,3-d]imidazol-1-yl]methyl}-phenyl}-cyclohex-1-yl}carbonyl}-phenylglycinamid Hydrochlorid**

Die Verbindung von Beispiel 3 wurde in einer möglichst geringen Menge Dichlormethan vollständig in Lösung gebracht und mit etwa 2 Äquivalenten 1M-HCl in Dietylether behandelt. Der entstandene Niederschlag wurde abgesaugt [Fp. 158°C (Zers.)].

### Beispiel 5

**(*S*)-N-{{(1*R*, 2*R*)-2-{4-{[2-(4-Methyl-piperazin-1-yl)-benzimidazol-1-yl]methyl}phenyl}-cyclohex-1-yl}carbonyl}-phenylglycinamid**
a) (1*R*, 2*R*)-2-{4-[(2-Chloro-benzimidazol-1-yl)methyl]-phenyl}-cyclohexan-1-carbonsäure-*tert*-butylester 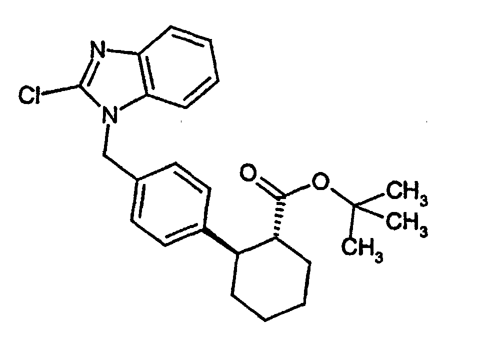
   Entsprechend allgemeiner Vorschrift [A] wurde die Titelverbindung aus 2-Chlorobenzimidazol und der Verbindung aus Beispiel 2A hergestellt [R_{*f*} (Cyclohexan:Essigester = 1:1) = 0,85].
b) (1*R*, 2*R*)-2-{4-{[2-(4-Methyl-piperazin-1-yl)-benzimidazol-1-yl]methyl}phenyl}-cyclohexan-1-carbonsäure 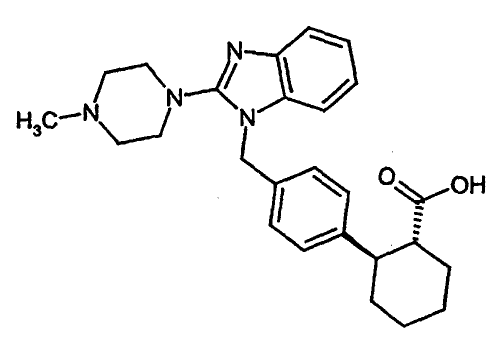
   Eine Lösung der Verbindung von Beispiel 5a (34,0 g, 56,0 mmol) in N-Methylpiperazin (77,7 mL, 700 mmol) wurde über Nacht auf 100°C erhitzt, anschliessend eingeengt und chromatographiert (Kieselgel, Dichlormethan:Methanol=20:1, bis 10:1, R_{*f*}(10:1)=0,32). Es wurden 32,0 g (1*R*, 2*R*)-2-{4-{[2-(4-Methyl-piperazin-1-yl)-benzimidazol-1-yl]methyl}-phenyl}-cyclohexan-1-carbonsäure -*tert*-butylester erhalten, die über Nacht bei Raumtemperatur mit Salzsäure (180 mL, 6 M) zur Reaktion gebracht wurden. Waschen des Reaktionsgemisches bei pH=7 mit Dichlormethan, Trocknen der organischen Phase über Magnesiumsulfat und Chromatographie (Kieselgel, Dichlormethan:Methanol 5:1, R_{*f*} =0,13) lieferte 19 g (78% der Theorie über 2 Stufen) der Titelverbindung. MS (ESI)=433 (M+H⁺). ¹H-NMR (DMSO-d₆): 1,35-1,5 (4 H, m); 1,65-1,8 (3 H, m); 1,9-2,0 (1 H, m); 2,2 (3 H, s); 2,4-2,5 (5 H, m); 2,6-2,7 (1 H, m); 3,15 (4 H, ψ t); 3,4 (1 H, very br s); 5,2 (2 H, s); 7,0-7,2 (7 H, m); 7,4,(1 H, d).
c) *(S)*-N-{{(1*R*, 2*R*)-2-{4-{[2-(4-Methyl-piperazin-1-yl)-benzimidazol-1-yl]methyl}-phenyl}-cyclohex-1-yl}carbonyl}-phenylglycinamid 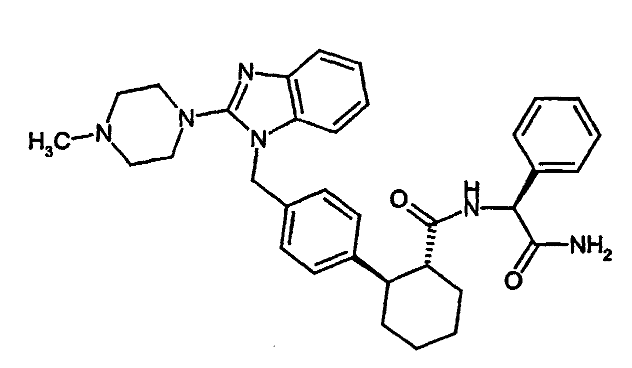
   Eine Suspension der Verbindung von Beispiel 5b (19 g, 43,9 mmol), *(S)-*Phenylglycinamid-Hydrochlorid (8,61 g, 46,1 mmol), 1-Hydroxybenztriazol (7,68 g, 48,3 mmol), EDC-Hydrochlorid (9,68 g, 50,5 mmol) und Triethylamin (24,5 mL, 175,7 mmol) in Dichlormethan (1000 mL) wurde über das Wochenende bei Raumtemperatur gerührt. Nach Zugabe von Wasser wurde mit Dichlormethan/-Methanol extrahiert, über Magnesiumsulfat getrocknet und eingeengt. Der leicht gelbliche Festkörper wurde in Dichlormethan/Methanol (10:1, 220 mL) verrührt, die saubere Titelverbindung wurde abgenutscht und bei 40°C am Vakuum getrocknet (14,5 g, 59%). R_{*f*} (Dichlormethan:Methanol 10:1)=0,30. MS (DCI, NH₃)=565 (M+H⁺). ¹H-NMR (DMSO-d₆): 1,2-1,5 (4 H, m); ); 1,6-1,85 (4 H, m); 2,2 (3 H, s); 2,45 (4 H, ψ t); 2,65 (1 H, br t); 2,8 (1 H, td); 3,15 (4 H, ψ t); 5,15 (1 H, d); 5,2 (2 H, s); 6,9 (2 H, d); 6,95-7,2 (11 H, m); 7,45 (1 H, d); 7,6 (1 H, br s); 8,0 (1 H, d).

### Beispiel 6

**(*S*)-N-{{(1*R*, 2*R*)-2-{4-{[2-(4-Methyl-piperazin-1-yl)-benzimidazol-1-yl]methyl}phenyl}-cyclohex-1-yl}earbonyl}-phenylglycinamid Hydrochlorid**

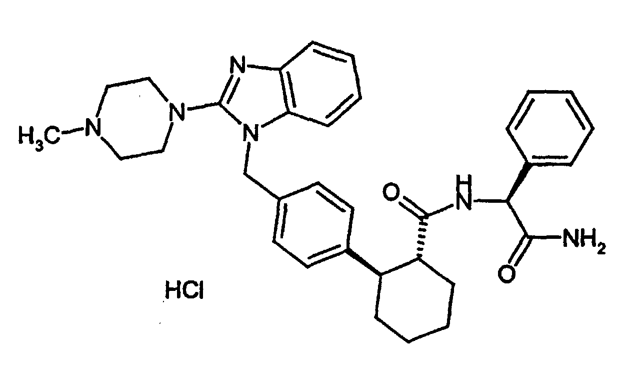

Die Verbindung von Beispiel 5 (100mg, 0,177 mmol) wurde in Dichlormethan/Methanol (2,5:1; 5mL) gelöst und mit 1M-HCl/Diethylether (0,177 mmol) versetzt, 5 min gerührt und dann in der Kälte am Vakuum eingeengt. Die Titelverbindung wurde als farbloses Pulver (106 mg) erhalten. Fp 200°C (Zers.).

Die in der folgenden Tabelle 1 aufgeführten Beispiele 7 bis 10 wurden analog Beispiel 5 unter Verwendung der entsprechend substituierten Piperazine hergestellt.

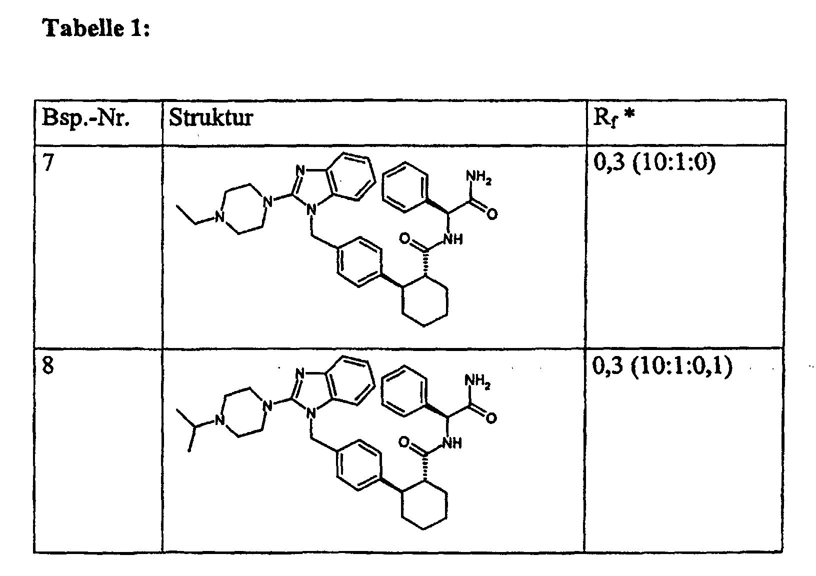

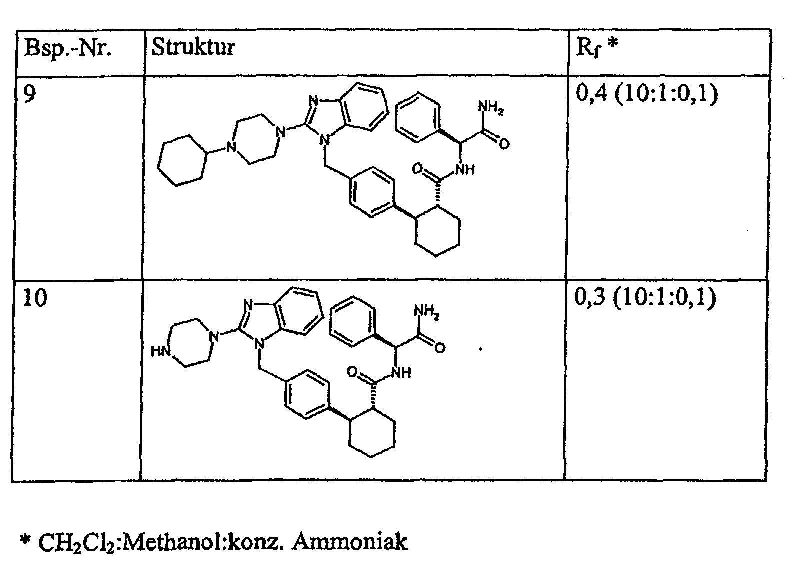

Die in der folgenden Tabelle 2 aufgeführten Beispiele 11 und 12 werden ausgehend von der Verbindung aus Beispiel 6A nach den allgemeinen Vorschriften A, B und C hergestellt.

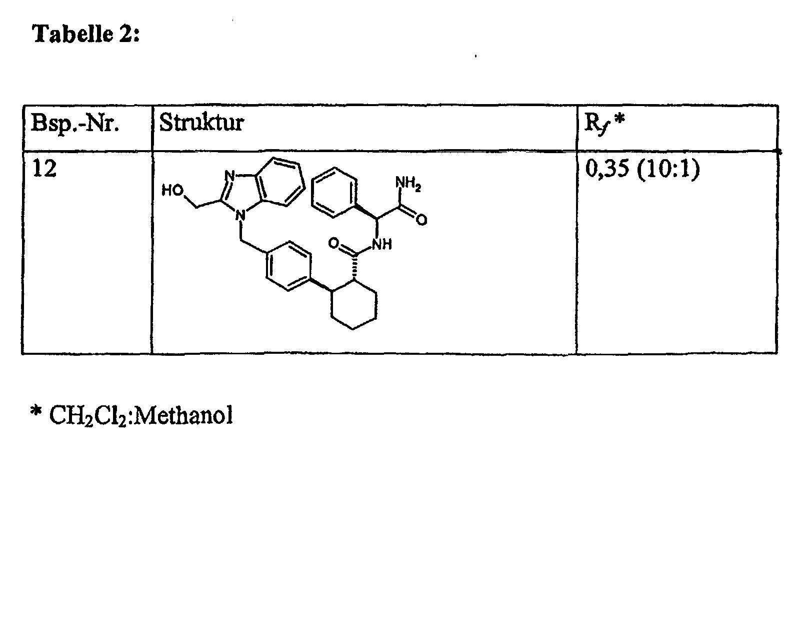

### Beispiel 13

**(*S*)-N-{{(1*R*, 2*R*)-2-{4-{[2-(2-Hydroxyethoxy)methyl]-benzimidazol-1-yl}-methyl}phenyl}-cyclohex-1-yl}carbonyl}-phenylglycinamid**

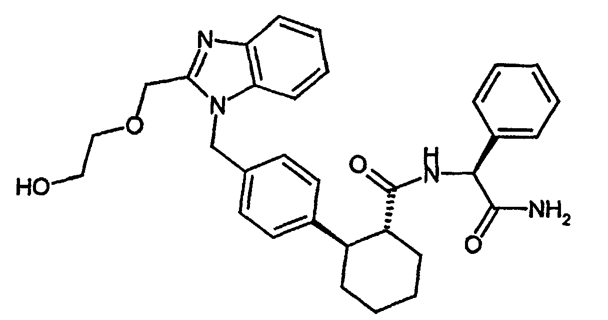

Ausgehend von der Verbindung des Beispiels 7A, das analog zu Beispiel 6A mit TBDMS-Chlorid silyliert wird und anschließend nach allgemeiner Vorschrift A, B und C umgesetzt wird, wird die Titelverbindung erhalten.

R_{*f*}(Dichlormethan:Methanol 20:1)=0,20.

MS (ESI)=541 (M+H⁺). ¹H-NMR (DMSO-d₆): 1,2-1,5 (4 H, m); ); 1,6-1,9 (4 H, m); 2,6-2,7 (1 H, m); 2,75-2,85 (1 H, m); 3,5 (4 H, s); 4,65 (1 H, br s); 4,6 (2 H, s); 5,15 (1 H, d); 5,55 (2 H, s); 6,9 (2 H, d); 6,95-7,2 (10 H, m); 7,45 (1 H, m); 7,6 (1 H, s); 7,65 (1 H, m); 8,05 (1 H, d).

Die in der folgenden Tabelle 3 aufgeführten Beispiele 14 bis 16 werden analog Beispiel 13 aus den entsprechenden Edukten hergestellt.

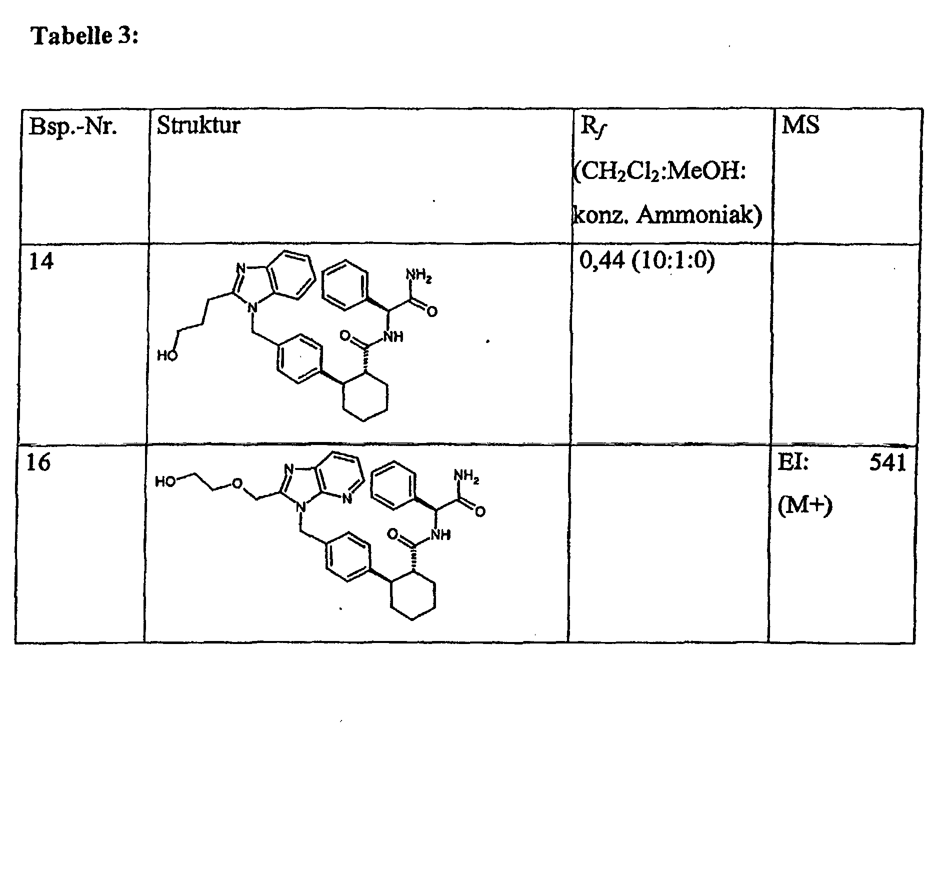

## Patentansprüche

1. Verbindungen der allgemeinen Formel (I), 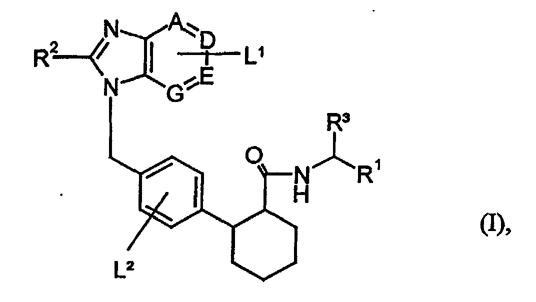 wobei
A, D und E jeweils für die CH-Gruppe stehen,
G für ein Stickstoffatom oder für die CH-Gruppe steht,
L¹ und L² für Wasserstoff stehen,
R¹ für einen Rest der Formel -CO-NR⁴R⁵ steht,
worin
R⁴ und R⁵ Wasserstoff bedeuten,
R² für (C₁-C₄)-Alkyl steht, das gegebenenfalls durch ein Sauerstoffatom unterbrochen ist, oder
für einen 4-R⁷-Piperazin-1-ylrest steht
wobei (C₁-C₄)-Alkyl, das gegebenenfalls durch ein Sauerstoffatom unterbrochen ist, durch eine Hydroxyl-Gruppe oder durch einen Rest der Formel -NR⁸R⁹ substituiert ist,
worin
R⁷ Wasserstoff, (C₁-C₄)-Alkyl oder (C₃-C₆)-Cycloalkyl bedeuten,
R⁸ und R⁹ gleich oder verschieden sind und Wasserstoff, (C₁-C₄)-Alkyl oder (C₃-C₆)-Cycloalkyl bedeuten, oder
R⁸ und R⁹ gemeinsam mit dem Stickstoffatom einen Morpholinrest bilden,
und
R³ für einen Phenylrest steht,
und deren Salze.

2. (*S*)-N-{{(1*R*, 2*R*)-2-{4-{[2-(4-Methyl-piperazin-1-yl)-benzimidazol-1-yl]methyl}-phenyl}-cyclohex-1-yl}carbonyl}-phenylglycinamid 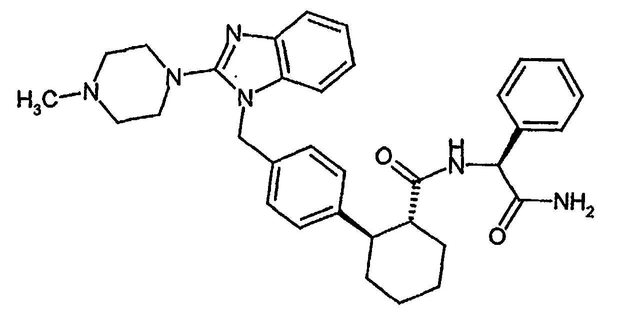 und dessen Salze.

3. Verfahren zur Herstellung von Verbindungen der allgemeinen Formel (I) gemäß Anspruch 1 bis 4, **dadurch gekennzeichnet, daß** man
[A] Verbindungen der allgemeinen Formel (II) 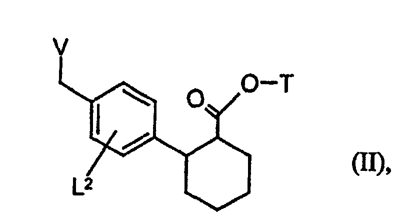 in welcher
L² die in Anspruch 1 angegebene Bedeutung hat,
T für (C₁-C₄)-Alkyl, vorzugsweise für Methyl oder tert.Butyl steht,
und
V für eine geeignete Abgangsgruppe, wie beispielsweise Halogen, Mesylat oder Tosylat, vorzugsweise für Brom steht,
zunächst durch Umsetzung mit Verbindungen der allgemeinen Formel (III) 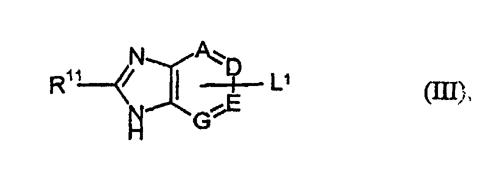 in welcher
A, D, E, G und L¹ die in Anspruch 1 angegebene Bedeutung haben
und
R¹¹ die in Anspruch 1 aufgeführte Bedeutung von R² hat, wobei Aminound Hydroxyfunktionen gegebenenfalls durch geeignete Amino- bzw. Hydroxyschutzgruppen blockiert sind, in inerten Lösemitteln, in Abhängigkeit der Definition von R¹¹ gegebenenfalls in Anwesenheit einer Base in die Verbindungen der allgemeinen Formel (IV) 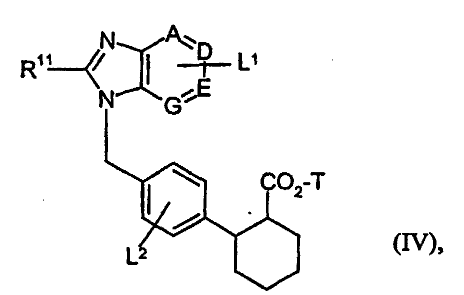
in welcher
R¹¹, A, D, E, G, L¹, L², und T die oben angegebene Bedeutung haben,
überführt,
in einem nächsten Schritt mit Säuren oder Basen in die entsprechenden Carbonsäuren der allgemeinen Formel (V) 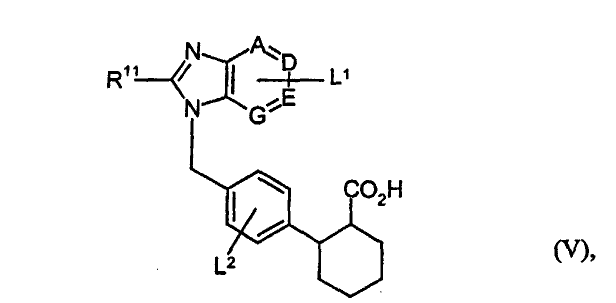 in welcher
R¹¹, A, D, E, G, L¹ und L² die oben angegebene Bedeutung haben,
überführt,
und abschließend nach bekannten Methoden mit Verbindungen der allgemeinen Formel (VI) 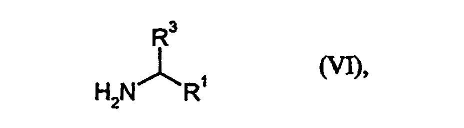 in welcher
R¹ und R³ die in Anspruch 1 angegebene Bedeutung haben,
in inerten Lösemitteln umsetzt,
und gegebenenfalls im Fall daß R¹¹ eine der oben aufgeführten Schutzgruppen trägt, diese entweder bei der Hydrolyse zu den Säuren (IV) -> (V) oder nach Umsetzung mit den Verbindungen der allgemeinen Formel (VI) nach üblichen Methoden abspaltet,
oder
[B] im Fall, daß R² für einen direkt über ein Stickstoffatom an den Imidazolring gebundenen, gesättigten Heterocyclus steht,
zunächst die oben aufgeführten Verbindungen der allgemeinen Formel (II) mit Verbindungen der allgemeinen Formel (IIIa) 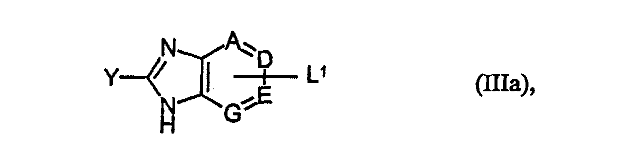
in welcher
A, D, E, G und L¹ die in Anspruch 1 angegebene Bedeutung haben
und
Y für Halogen oder Mesyl , vorzugsweise für Chlor, Brom oder Mesyl steht,
in inerten Lösemitteln in die entsprechenden Verbindungen der Formel (VII) 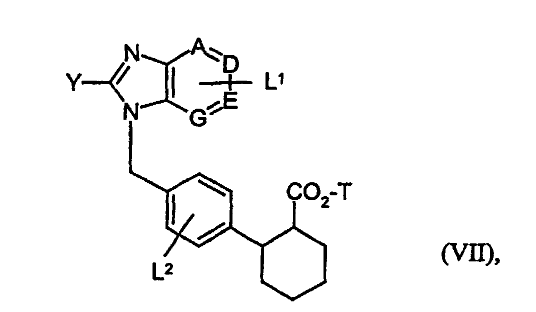 in welcher
Y, A, D, E, G, L¹, L² und T die oben angegebene Bedeutung haben,
überführt,
in einem nächsten Schritt mit Verbindungen der allgemeinen Formel (VIII)
HNR¹²R¹³ (VIII)
in welcher
R¹² und R¹³ zusammen mit dem Stickstoffatom einen Heterocyclus gemäß der Definition von R² bilden,
zu Verbindungen der allgemeinen Formel (IX) 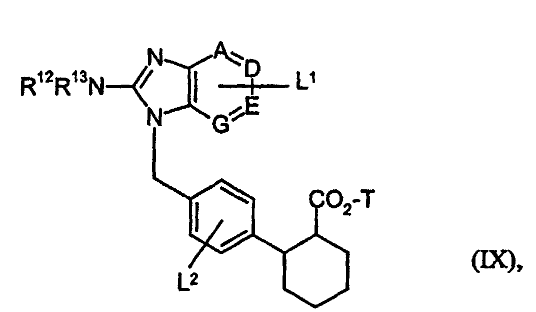 in welcher
A, D, E, G, L¹, L², R¹², R¹³ und T die oben angegebene Bedeutung haben,
umsetzt,
in den nächsten Schritten, wie unter [A] beschrieben durch Hydrolyse in die entsprechenden Carbonsäuren der allgemeinen Formel (X) 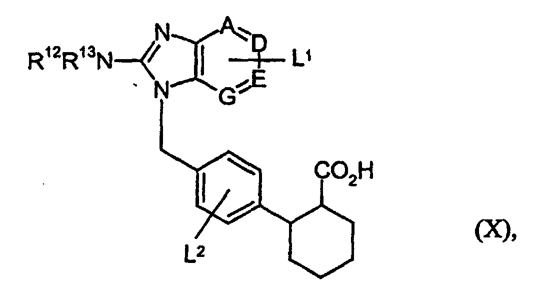 in welcher
A, D, E, G, L¹, L², R¹² und R¹³ die oben angegebene Bedeutung haben,
überführt,
und abschließend nach bekannten Methoden zur Herstellung von Amiden aus Carbonsäuren und Aminen mit den Verbindungen der allgemeinen Formel (VI) umsetzt und gegebenenfalls durch Umsetzung mit einer Säure in die entsprechenden Salze überführt.

4. Verbindungen der allgemeinen Formel (IV) 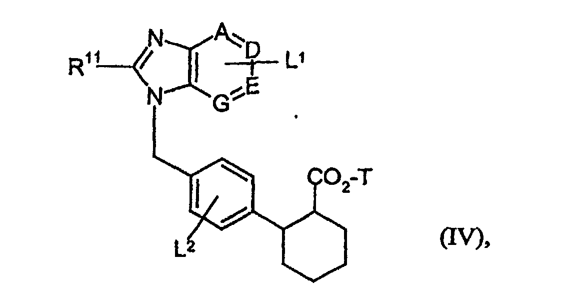 worin
A, D, E, G, L¹, L², R¹¹ und T die in den Ansprüchen 1 und 3 angegebene Bedeutung haben,
und deren Salze.

5. Verbindungen der allgemeinen Formel (V) 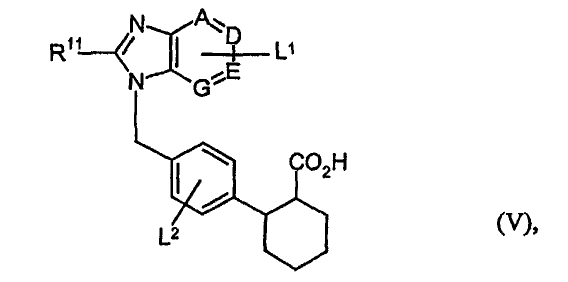 worin
A, D, E, G, L¹, L² und R¹¹ die in den Ansprüchen 1 und 3 angegebene Bedeutung haben,
und deren Salze.

6. Verbindungen der allgemeinen Formel (VII) 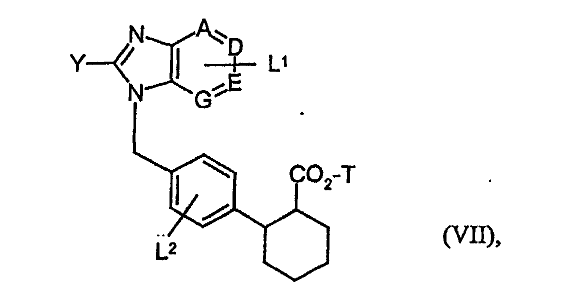 worin
A, D, E, G, L¹, L², Y und T die in den Ansprüchen 1 und 3 angegebene Bedeutung haben,
und deren Salze.

7. Verbindungen der allgemeinen Formel (IX) 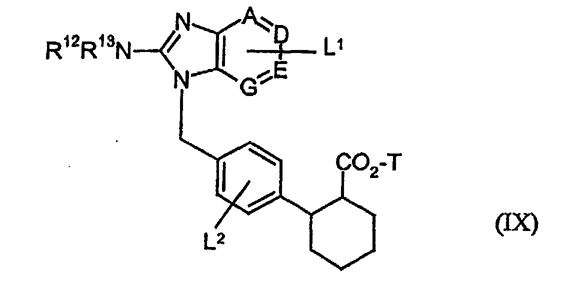 worin
A, D, E, G, L¹, L², R¹², R¹³ und T die in den Ansprüchen 1 und 3 angegebene Bedeutung haben,
und deren Salze.

8. Verbindungen der allgemeinen Formel (X) 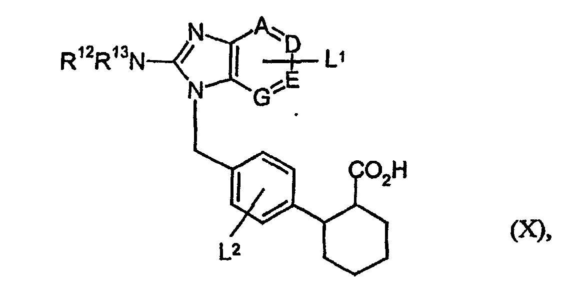 worin
A, D, E, G, L¹, L², R¹¹ und R¹² die in den Ansprüchen 1 und 3 angegebene Bedeutung haben,
und deren Salze.

9. Arzneimittel enthaltend eine Verbindung der allgemeinen Formel (I) gemäß einem der Ansprüche 1 und 2 in Zusammenmischung mit mindestens einem pharmazeutisch verträglichen, im wesentlichen ungiftigen Träger oder Exzipienten.

10. Verbindungen gemäß einem der Ansprüche 1 und 2 zur Verwendung als Arzneimittel in der Behandlung von Menschen und Tieren.

11. Verwendung von Verbindungen gemäß einem der Ansprüche 1 und 2 zur Herstellung von Arzneimitteln zur Behandlung und/oder Prophylaxe von ischämischen Hirnerkrankungen.

12. Verwendung von Verbindungen gemäß einem der Ansprüche 1 und 2 zur Herstellung von Arzneimitteln zur Behandlung und/oder Prophylaxe von Hirnschlag, Reperfusionsschäden oder Hirntrauma.

## Claims

1. Compounds of the general formula (I) 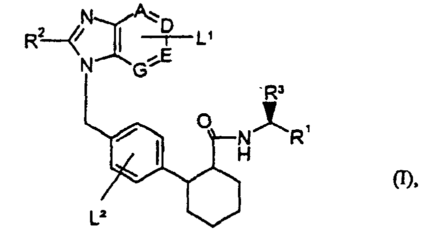 where
A, D and E each represent the CH group,
G represents a nitrogen atom or represents the CH group,
L¹ and L² represent hydrogen,
R¹ represents a radical of the formula -CO-NR⁴R⁵,
in which
R⁴ and R⁵ denote hydrogen,
R² represents (C₁-C₄)-alkyl which is optionally interrupted by one oxygen atom, or
represents a 4-R⁷-piperazin-1-yl radical
where (C₁-C₄)-alkyl which is optionally interrupted by one oxygen atom is substituted by a hydroxyl group or by a radical of the formula -NR⁸R⁹
in which
R⁷ denotes hydrogen, (C₁-C₄)-alkyl or (C₃-C₆)-cycloalkyl,
R⁸ and R⁹ are identical or different and denote hydrogen, (C₁-C₄)-alkyl or (C₃-C₆)-cycloalkyl,
or
R⁸ and R⁹ together with the nitrogen atom form a morpholine radical,
and
R³ represents a phenyl radical,
and their salts.

2. (*S*)-N-{{ (1*R*, 2*R*)-2-{4-{[2-(4-Methyl-piperazin-1-yl)-benzimidazol-1-yl]methyl}-phenyl}-cyclohex-1-yl}carbonyl}-phenylglycinamide 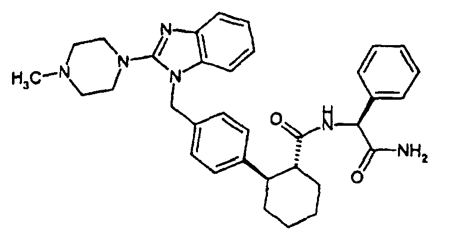 and its salts.

3. Process for preparing compounds of the general formula (I) according to Claims 1 to 4, **characterized in that**
[A] compounds of the general formula (II) 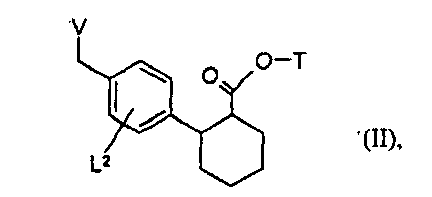 in which
L² is as defined in Claim 1,
T represents (C₁-C₄)-alkyl, preferably methyl or tert-butyl,
and
V represents a suitable leaving group, such as, for example, halogen, mesylate or tosylate, preferably bromine,
are initially converted by reaction with compounds of the general formula (III) 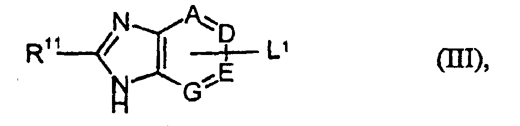 in which
A, D, E, G and L¹ are as defined in Claim 1
and
R¹¹ has the meaning of R² given in Claim 1, where amino and hydroxyl functions are optionally blocked by typical amino or hydroxyl protective groups,
in inert solvents, depending on the definition of R¹¹ optionally in the presence of a base, into the compounds of the general formula (IV) 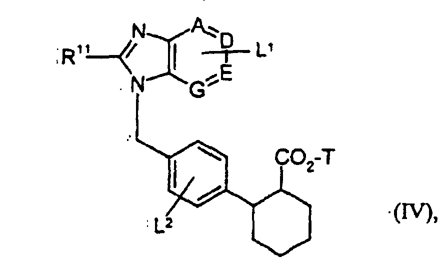 in which
R¹¹, A, D, E, G, L¹, L² and T are as defined above,
are converted in a subsequent step using acids or bases into the corresponding carboxylic acids of the general formula (V) 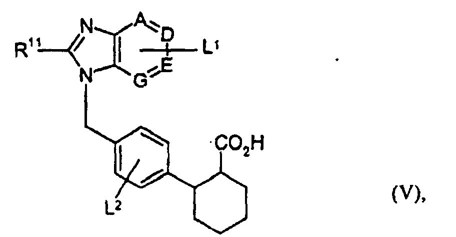 in which
R¹¹, A, D, E, G, L¹ and L² are as defined above,
and are subsequently reacted by known methods with compounds of the general formula (VI) 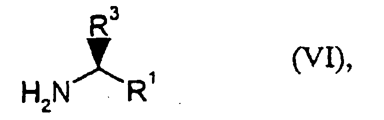 in which
R¹ and R³ as defined in Claim 1
in inert solvents,
and, if R¹¹ carries one of the abovementioned protective groups, this is optionally removed by customary methods either in the hydrolysis to the acids (IV) -> (V) or after the reaction with the compounds of the general formula (VI),
or
[B] if R² represents a saturated heterocycle which is attached directly via a nitrogen atom to the imidazole ring,
the abovementioned compounds of the general formula (II) are initially converted with compounds of the general formula (IIIa) 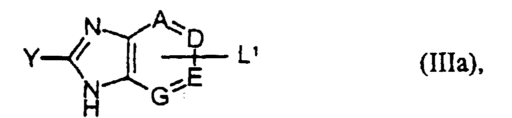 in which
A, D, E, G and L¹ are as defined in Claim 1
and
Y represents halogen or mesyl, preferably chlorine, bromine or mesyl,
in inert solvents into the corresponding compounds of the formula (VII) 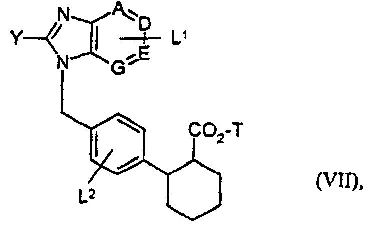 in which
Y, A, D, E, G, L¹, L² and T are as defined above,
are reacted in a subsequent step with compounds of the general formula (VIII)
HNR¹²R¹³ (VIII)
in which
R¹² and R¹³ together with the nitrogen atom form a heterocycle according to the definition of R²
to give compounds of the general formula (IX) 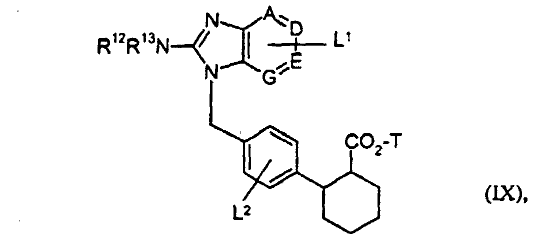 in which
A, D, E, G, L¹, L², R¹², R¹³ and T are as defined above,
are, in the subsequent steps, converted as described under [A] by hydrolysis into the corresponding carboxylic acids of the general formula (X) 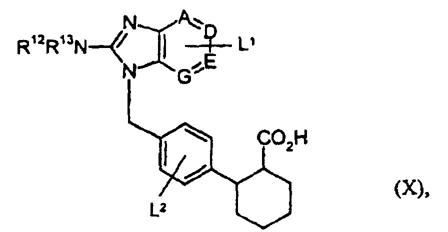 in which
A, D, E, G, L¹, L², R¹² and R¹³ are as defined above,
and these compounds are subsequently reacted with the compounds of the general formula (VI) according to known methods for preparing amides from carboxylic acids and amines and optionally converted into the corresponding salts by reaction with an acid.

4. Compounds of the general formula (IV) 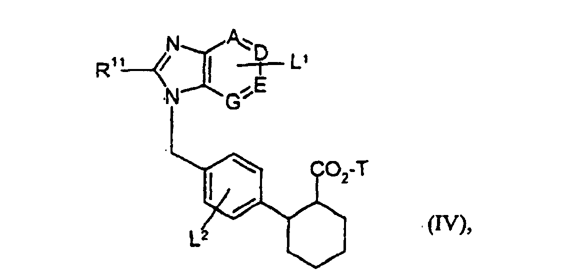 in which
A, D, E, G, L¹, L², R¹¹ and T are as defined in Claims 1 and 3
and their salts.

5. Compounds of the general formula (V) 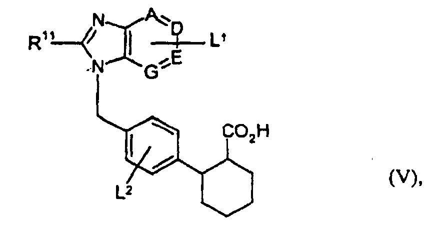 in which
A,D, E, G, L¹, L² and R¹¹ are as defined in Claims 1 and 3
and their salts.

6. Compounds of the general formula (VII) 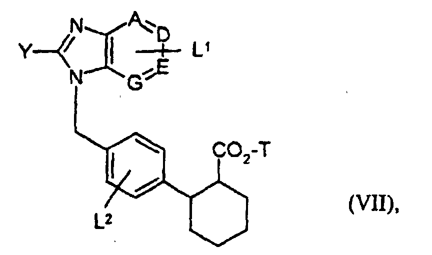 in which
A, D, E, G, L¹, L², Y and T are as defined in Claims I and 3
and their salts.

7. Compounds of the general formula (IX) 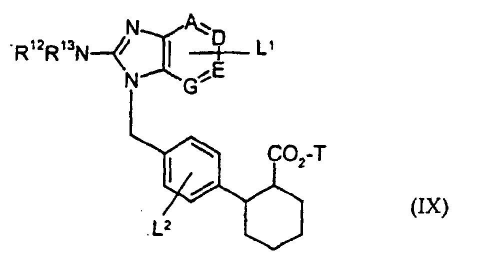 in which
A, D, E, G, L¹, L², R¹², R¹³ and T are as defined in Claims 1 and 3
and their salts.

8. Compounds of the general formula (X) 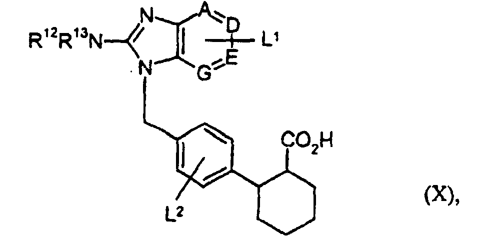 in which
A, D, E, G, L¹, L², R¹¹ and R¹² are as defined in Claims 1 and 3
and their salts.

9. Medicaments, comprising a compound of the general formula (I) according to either of Claims 1 and 2 in admixture with at least one pharmaceutically acceptable, essentially non-toxic carrier or excipient.

10. Compounds according to either of Claims 1 and 2 for use as medicament in the treatment of humans and animals.

11. Use of compounds according to either of Claims 1 and 2 for preparing medicaments for the treatment and/or prophylaxis of ischaemic brain disorders.

12. Use of compounds according to either of Claims 1 and 2 for preparing medicaments for the treatment and/or prophylaxis of stroke, reperfusion damage or brain trauma.

## Revendications

1. Composés de formule générale (I), 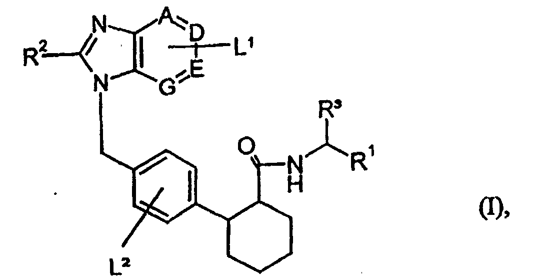 dans laquelle
A, D et E représentent chacun le groupe CH,
G représente un atome d'azote ou le groupe CH,
L¹ et L² représentent l'hydrogène,
R¹ est un reste de formule -CO-NR⁴R⁵,
dans lequel
R⁴ et R⁵ représentent l'hydrogène,
R² est un reste alkyle en C₁ à C₄, qui est éventuellement interrompu par un atome d'oxygène, ou représente
un reste 4-R⁷-pipérazin-1-yl,
le reste alkyle en C₁ à C₄, qui est éventuellement interrompu par un atome d'oxygène, étant substitué par un groupe hydroxyle ou par un reste de formule -NR⁸R⁹,
où
R⁷ représente l'oxygène, un reste alkyle en C₁ à C₄ ou cycloalkyle en C₃ à C₆,
R⁸ et R⁹ sont identiques ou différents et représentent l'hydrogène, un reste alkyle en C₁ à C₄ ou un reste cycloalkyle en C₃ à C₆,
ou bien
R⁸ et R⁹ forment conjointement avec l'atome d'azote un reste morpholine,
et
R³ est un reste phényle,
et leurs sels.

2. Le (*S*)-N-{{1*R*, 2*R*)-2-{4-{[2-(4-méthyl-pipérazin-1-yl)-benzimidazole-1-yl]méthyl}phényl}-cyclohex-1-yl}carbonyl}phénylglycinamide 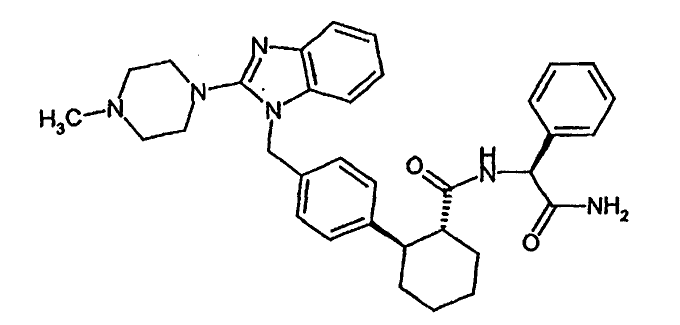 et ses sels.

3. Procédé de production de composés de formule générale (I) suivant la revendication 1, **caractérisé en ce que** :
[A] des composés de formule générale (II) 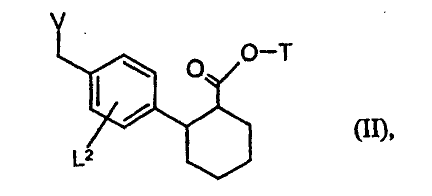 dans laquelle
L² a la définition indiquée dans la revendication 1,
T est un reste alkyle en C₁ à C₄, de préférence le reste méthyle ou tertiobutyle,
et
V représente un groupe partant approprié tel que, par exemple, halogène, mésylate ou tosylate, de préférence le brome,
sont transformés tout d'abord par réaction avec des composés de formule générale (III) 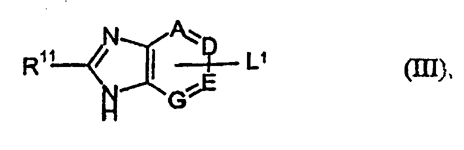 dans laquelle
A, D, E, G et L¹ ont la définition indiquée dans la revendication 1
et
R¹¹ a la définition indiquée pour R² dans la revendication 1, les fonctions amino et hydroxy étant éventuellement bloquées par des groupes protecteurs respectifs amino et hydroxy appropriés,
dans des solvants inertes, selon la définition de R¹¹, éventuellement en présence d'une base, en les composés de formule générale (IV) 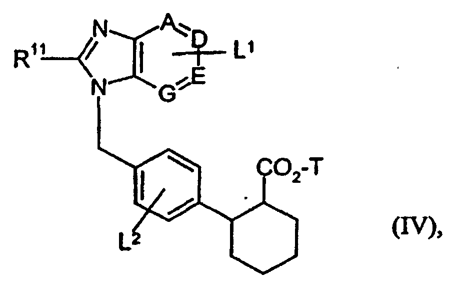 dans laquelle
R¹¹, A, D, E, G, L¹, L² et T ont la définition indiquée ci-dessus,
qui sont transformés dans une étape subséquente, avec des acides ou des bases, en les acides carboxyliques correspondants de formule générale (V) 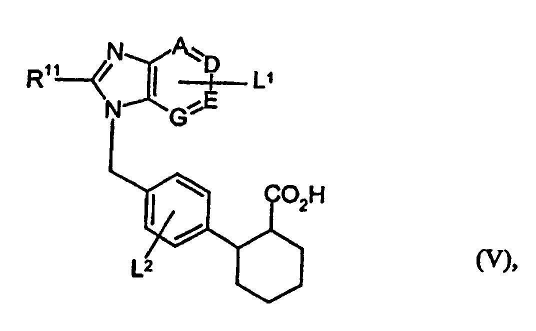 dans laquelle
R¹¹, A, D, E, G, L¹ et L² ont la définition indiquée ci-dessus,
que l'on fait ensuite réagir selon des modes opératoires connus avec des composés de formule générale (VI) 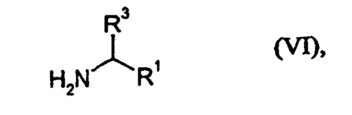 dans laquelle
R¹ et R³ ont la définition indiquée dans la revendication 1,
dans des solvants inertes,
et le cas échéant, au cas où R¹¹ porte l'un des groupes protecteurs indiqués ci-dessus, ce groupe est éliminé soit lors de l'hydrolyse en acides (IV)→(V) ou après réaction avec les composés de formule générale (VI) selon des modes opératoires classiques,
ou bien
[B] au cas où R² représente un hétérocycle saturé lié directement par un atome d'azote au noyau d'imidazole,
les composés indiqués ci-dessus de formule générale (II) sont tout d'abord transformés avec des composés de formule générale (IIIa) 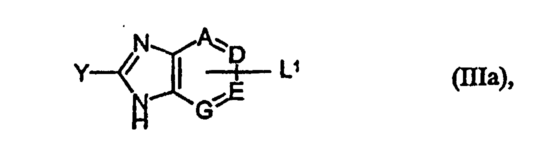
dans laquelle
A, D, E, G et L¹ ont la définition indiquée dans la revendication 1
et
Y est un halogène ou un reste mésyle, de préférence le chlore, le brome ou le reste mésyle,
dans des solvants inertes, en les composés correspondants de formule (VII) 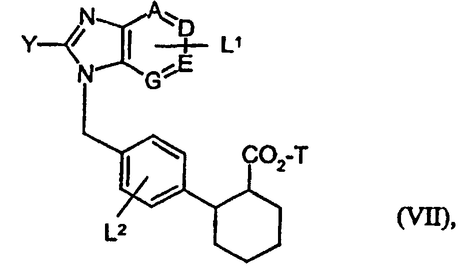 dans laquelle
Y, A, D, E, G, L¹, L² et T ont la définition indiquée ci-dessus,
qui sont amenés à réagir dans une étape subséquente avec des composés de formule générale (VIII)
HNR¹²R¹³ (VIII)
dans laquelle
R¹² et R¹³ forment conjointement avec l'atome d'azote un hétérocycle conformément à la définition de R²,
pour former des composés de formule générale (IX) 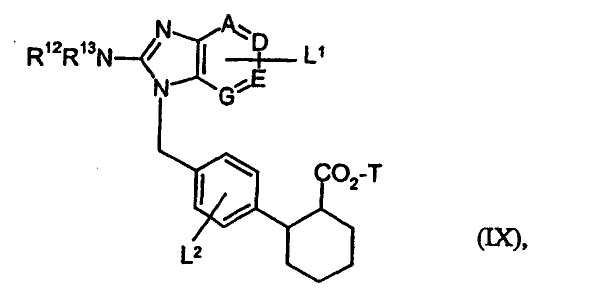 dans laquelle
A, D, E, G, L¹, L², R¹², R¹³ et T ont la définition indiquée ci-dessus,
qui sont transformés dans l'étape suivante, par hydrolyse comme décrit en [A], en les acides carboxyliques correspondants de formule générale (X) 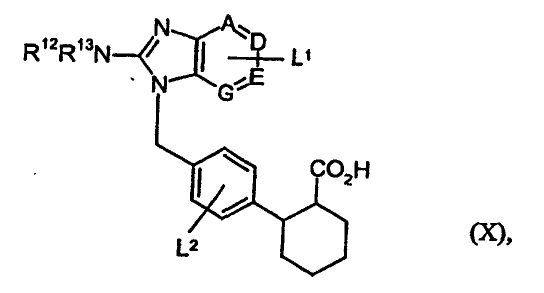 dans laquelle
A, D, E, G, L¹, L², R¹² et R¹³ ont la définition indiquée ci-dessus,
qui sont finalement amenés à réagir, selon des modes opératoires connus pour la préparation d'amides à partir d'acides carboxyliques et d'amines, avec les composés de formule générale (VI) et le cas échéant transformés en les sels correspondants par réaction avec un acide.

4. Composés de formule générale (IV) 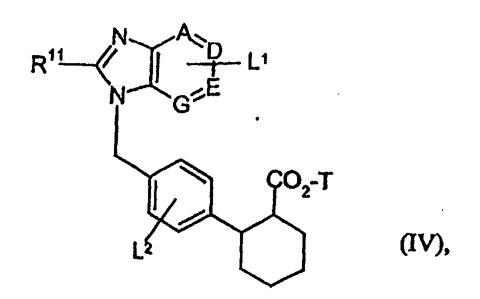 dans laquelle
A, D, E, G, L¹, L², R¹¹ et T ont la définition indiquée dans les revendications 1 et 3,
et leurs sels.

5. Composés de formule générale (V) 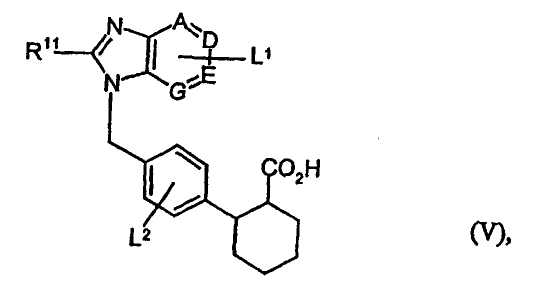 dans laquelle
A, D, E, G, L¹, L² et R¹¹ ont la définition indiquée dans les revendications 1 et 3,
et leurs sels.

6. Composés de formule générale (VII) 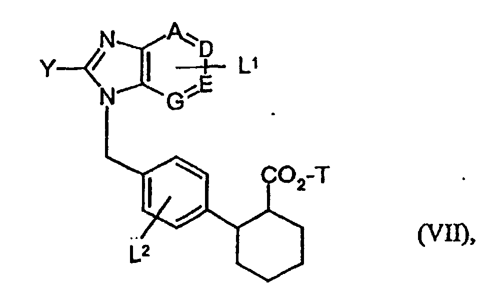 dans laquelle
A, D, E, G, L¹, L², Y et T ont la définition indiquée dans les revendications 1 et 3,
et leurs sels.

7. Composés de formule générale (IX) 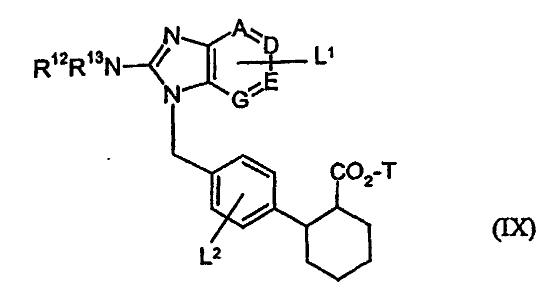 dans laquelle
A, D, E, G, L¹, L², R¹², R¹³ et T ont la définition indiquée dans les revendications 1 et 3,
et leurs sels.

8. Composés de formule générale (X) 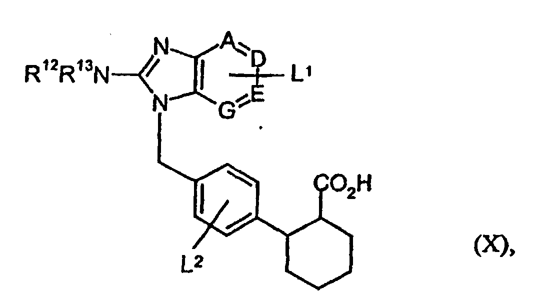 dans laquelle
A, D, E, G, L¹, L², R¹¹ et R¹² ont la définition indiquée dans les revendications 1 et 3,
et leurs sels.

9. Médicament contenant un composé de formule générale (I) suivant l'une des revendications 1 et 2 en association avec au moins un support ou excipient principalement non toxique, acceptable du point de vue pharmaceutique.

10. Composés suivant l'une des revendications 1 et 2, destinés à être utilisés comme médicament dans le traitement d'êtres humains et d'animaux.

11. Utilisation de composés suivant l'une des revendications 1 et 2 pour la préparation de médicaments destinés au traitement et/ou à la prophylaxie d'affections cérébrales ischémiques.

12. Utilisation de composés suivant l'une des revendications 1 et 2 pour la préparation de médicaments destinés au traitement et/ou à la prophylaxie de l'apoplexie cérébrale, de dommages en rapport avec la reperfusion ou de lésions cérébrales.
